# FASCICULE DE BREVET EUROPEEN

(11) **EP 1 735 344 B1**
(45) Date de publication et mention de la délivrance du brevet: **14.12.2011**
(21) Numéro de dépôt: 05757060.8
(22) Date de dépôt: 13.04.2005
(51) Int. Cl.: C07K 14/705, C12N 15/62

(54) **COMPOSITION DE VACCIN COMPRENANT UN LIGAND CMH DE CLASSE II COUPLE A UN ANTIGENE, PROCEDE DE PREPARATION ET UTILISATIONS**
IMPFSTOFFZUSAMMENSETZUNG ENTHALTEND EINEN KLASSE-II-MHC-LIGANDEN GEKOPPELT MIT EINEM ANTIGEN, VERFAHREN ZU DESSEN HERSTELLUNG UND DESSEN VERWENDUNG
VACCINE COMPOSITION COMPRISING A CLASS II MHC LIGAND COUPLED WITH AN ANTIGEN, METHOD FOR THE PREPARATION AND THE USE THEREOF

(30) Priorité: 13.04.2004 FR 0403848
(43) Date de publication de la demande: 27.12.2006
(73) Titulaire: Immutep, 91400 Orsay (FR)
(72) Inventeur: TRIEBEL, Frédéric, F-78000 Versailles (FR)
(74) Mandataire: Thornton, Neil
(86) Numéro de dépôt international: PCT/FR2005/000894
(87) Numéro de publication internationale: WO 2005/103079

(56) Documents cités:
- EP-A- 0 893 507
- WO-A-98/23741
- LIU DAI-WEI ET AL: "Recombinant adeno-associated virus expressing human papillomavirus type 16 E7 peptide DNA fused with heat shock protein DNA as a potential vaccine for cervical cancer" JOURNAL OF VIROLOGY, vol. 74, no. 6, mars 2000 (2000-03), pages 2888-2894, XP002346954 ISSN: 0022-538X
- EL MIR S ET AL: "A soluble lymphocyte activation gene-3 molecule used as a vaccine adjuvant elicits greater humoral and cellular immune responses to both particulate and soluble antigens." JOURNAL OF IMMUNOLOGY (BALTIMORE, MD. : 1950), (2000 JUN 1) 164 (11) 5583-9. JOURNAL CODE: 2985117R. ISSN: 0022-1767., juin 2000 (2000-06), XP002291776
- KOST T A ET AL: "HUMAN IMMUNODEFICIENCY VIRUS INFECTION AND SYNCYTIUM FORMATION IN HELA CELLS EXPRESSING GLYCOPHOSPHOLIPID-ANCHORED CD4" JOURNAL OF VIROLOGY, vol. 65, no. 6, juin 1991 (1991-06), pages 3276-3283, XP009035008 ISSN: 0022-538X
- PARK SEUNG-WON ET AL: "Defective interfering HIV-1 pseudotypes carrying chimeric CD4 protein" JOURNAL OF BIOCHEMISTRY AND MOLECULAR BIOLOGY, vol. 34, no. 6, 30 novembre 2001 (2001-11-30), pages 566-572, XP002346955 ISSN: 1225-8687
- CARRERE-KREMER SEVERINE ET AL: "Subcellular localization and topology of the p7 polypeptide of hepatitis C virus" JOURNAL OF VIROLOGY, vol. 76, no. 8, avril 2002 (2002-04), pages 3720-3730, XP002346956 ISSN: 0022-538X

## Description

La présente invention concerne le domaine des vaccins thérapeutiques. L'invention se rapporte à un vaccin capable d'accroître l'action immunogène d'un antigène grâce au couplage de cet antigène à un ligand du CMH de classe II, tel que LAG-3 ou CD4. Elle concerne tout particulièrement un produit de couplage, notamment sous la forme d'une protéine de fusion, comprenant au moins un antigène spécifique de la pathologie pour laquelle on souhaite induire une immunisation et au moins un ligand du CMH de classe II.

Les ligands naturels des protéines du CMH de classe II tels que LAG-3, aussi désigné CD223, ou CD4 sont impliquées dans la reconnaissance immunitaire, notamment au niveau de l'interaction avec différentes cellules lymphoïdes comme les lymphocytes et les cellules présentatrices d'antigènes.

Il a été proposé dans la demande de brevet internationale PCT WO 99/04810 d'utiliser un ligand du CMH de classe II, comme LAG-3, à titre d'adjuvant pour la fabrication de vaccins destinés à l'immunothérapie des cancers.

Le document WO 98/23741 décrit une composition thérapeutique comprenant en plus d'un ou plusieurs mutants de LAG-3 un autre ingrédient actif, par exemple une toxine, comme la ricine ou l'anatoxine diphtérique, éventuellement lié par liaison chimique au mutant de LAG-3.

Le document EP 0 893 507 décrit une composition pharmaceutique comprenant une quantité efficace d'un ligand du CMH de classe II et une quantité efficace d'un antigène capable de stimuler le système immunitaire, préférablement via une réponse cellulaire T.

Le document Liu et al. (Liu et al. « Recombinant adeno-associated virus expressing human papillomavirus type 16E7 peptide DNA fused with heat shock protein DNA as a potential vaccine for cervical cancer », Journal of virology, vol. 74, no. 6, mars 2000, pages 2888-2894) décrit un produit de couplage constitué d'un épitope CTL de l'antigène E7 d'HPV (human papillomavirus) et d'une protéine HSP (heat shock protein).

Le document Park et al. (Park Seung-Won et al. « Defective interfering HIV-1 pseudotypes carrying chimeric CD4 protein », Journal of biochemistry and molecular biology, vol. 34, no. 6, pages 566-572) décrit un produit de couplage entre l'ectodomaine de CD4 et la protéine HIV-1 Env, HIV gp41.

Les travaux réalisés dans le cadre de la présente invention ont maintenant permis de mettre en évidence l'efficacité remarquable d'une immunisation par une protéine de fusion constituée de LAG-3 et d'un antigène. La Demanderesse a en effet observé une très forte réponse CD8 obtenue avec des doses très faibles d'un produit de couplage LAG-3-antigène *in vitro,* par rapport aux doses utilisées dans l'art antérieur pour des compositions de vaccins comprenant un antigène et la protéine LAG-3 à titre d'adjuvant.

Cette efficacité résulte de l'addition de l'effet adjuvant LAG-3Ig classique à un effet de ciblage de l'antigène (vectorisation) sur les cellules présentatrices (cellules dendritiques), qui permet l'internalisation de l'antigène lié par LAG-3 aux molécules du CMH de classe II. Un phénomène important de « cross-présentation » de l'antigène vers la voie de la présentation du CMH de classe I permet ainsi d'induire des réponses T CD8 alors que seules des réponses CD4 sont attendues avec un antigène exogène comme une protéine vaccinale.

En outre, les données expérimentales rapportées ci-après montre l'internalisation rapide à 37°C du produit de couplage LAG-3Ig/Ag par microscopie confocale (internalisation en 15 minutes).

La présente invention a donc pour objet un produit de couplage, de préférence de nature principalement protéique, constitué d'une première classe de protéines de type antigène choisie dans le groupe comprenant des antigènes viraux, des antigènes bactériens. des antigènes tumoraux, des antigènes de parasites et leurs mélanges et d'une seconde classe de protéines de type ligand du CMH de classe II, choisie dans le groupe comprenant hLAG-3, ses homologues. fragments et dérivés et les mélanges de ceux-ci. les deux classes de protéines étant couplées par une ou plusieurs liaisons stables dans les milieux biologiques, lesdites homologues, fragments et dérivés étant capables d'assurer une fixation avec une forte affinité sur le CMH de classe II des cellules dendritiques et l'internalisation de l'antigène de ladite première classe de protéine.

Par liaison stable dans les milieux biologiques, on entend sans limitations aussi bien une liaison covalente (par exemple une liaison amide ou un pont disulfure), ionique, hydrogène, de Van der Waals, hydrophobe et n'importe quelles combinaisons de celle-ci, ladite liaison permettant de maintenir l'intégrité du produit de couplage selon l'invention dans les milieux biologiques.

De préférence, les deux classes de protéines du produit de couplage selon l'invention sont liées par des liaisons hydrogènes ou des liaisons covalentes et de manière particulièrement préférée par des liaisons covalentes.

Selon une première forme de réalisation, un produit de couplage de l'invention est caractérisé par le fait que les deux classes de protéines sont liées par des liaisons hydrogènes.

Selon une seconde forme de réalisation, un produit de couplage de l'invention est caractérisé par le fait que les deux classes de protéines sont liées par covalence. Les deux classes de protéines peuvent être liées par covalence directement ou indirectement via un bras de liaison ou une molécule de liaison.

On entend par liaison covalente directe la mise en commun d'un ou plusieurs électrons des atomes de la première classe de protéines et de la seconde classe de protéines selon l'invention.

A titre d'exemple, de bras de liaison ou de molécules de liaison, on peut citer un polypeptide ou un acide aminé, un polysaccharide ou un monosaccharide, un polynucléotide ou un acide nucléique, un groupement alkyle, cyclo-alkyle ou aryle.

Une forme de réalisation avantageuse de l'invention est un produit de couplage qui se présente sous la forme d'une protéine de fusion où les deux classes de protéines sont liées directement ou indirectement par une ou plusieurs liaisons peptidiques. On entend par protéine de fusion, tout produit de couplage permettant la maturation et l'expression des deux classes de protéines selon l'invention dans une seule et même phase de lecture. Dans le cas de protéines de fusion, les antigène(s) et ligand(s) du CMH de classe II sont liés par covalence au niveau d'extrémités N et/ou C terminales.

Ainsi, différentes combinaisons de protéines recombinantes ont été préparées dans lesquelles hLAG-3 a été utilisé à titre de ligand du CMH sous sa forme 4 domaines Ig (D1D4) ou 2 domaines Ig (D1D2) et où les antigènes viraux E7 ou gag-nef utilisés à titre d'antigène ont été placés soit en N terminal, soit en C-terminal de hLAG-3Ig.

A titre d'exemple de produits de couplage où la première et la seconde classe de protéines sont liées par covalence, on peut citer ceux répondant à la formule (I) suivante :

[(Ag)ₙ(X)ₘ(Y)ₚ]_{q}

dans laquelle :
Ag représente l'antigène de la première classe de protéine, et n représente le nombre de molécules d'antigène dans le produit de couplage, n étant un nombre entier compris entre 1 et 5,
Y représente un ligand du CMH de classe II de la seconde classe de protéines, et p représente le nombre de ligand du CMH de classe II dans le produit de couplage, p étant un nombre entier compris entre 1 et 2,
X représente la liaison entre Ag et Y, et m représente le nombre de liaison entre Ag et Y dans le produit de couplage, m étant un nombre entier compris entre 1 et 5,
q est un nombre entier compris entre 1 et 5.

Dans le cas où les deux classes de protéines sont liées directement ou indirectement par covalence, X est choisi dans le groupe comprenant une liaison covalente, un bras de liaison ou une molécule de liaison, comme définis précédemment.

Ainsi, dans un produit de couplage selon l'invention, la première classe de protéine peut comprendre un seul antigène ou plusieurs antigènes identiques ou différents. De préférence, le produit de couplage selon l'invention comprend un seul antigène.

De même, dans un produit de couplage selon l'invention, la seconde classe de protéines peut comprendre un seul ligand du CMH de classe II ou plusieurs ligands du CMH de classe II identiques ou différents. De préférence, le produit de couplage selon l'invention comprend un seul ligand du CMH de classe II.

Dans le cas où le produit de couplage selon l'invention comprend plusieurs antigènes et/ou ligands du CMH de classe II :
- chaque classe de protéines peut être regroupée sous la forme d'un polymère, par exemple un dimère du ligand du CMH de classe II, lié par covalence à un monomomère ou polymère de l'antigène,
- les antigènes et/ou ligands du CMH de classe II sont alternés, et peuvent former des motifs répétés sous forme de polymères.

Les constructions protéiques ci-dessus peuvent être préparées sous forme de protéine de fusion en utilisant toutes techniques bien connues de l'homme du métier telles que la technique de l'ADN recombinant ou la synthèse chimique. La technique de l'ADN recombinant repose sur la préparation d'ADN recombinants comprenant les séquences nucléotidiques codant les constructions protéiques selon l'invention.

Dans le cas d'une préparation des produits de couplage selon l'invention par synthèse chimique, les antigène(s) et ligand(s) du CMH de classe II peuvent être aussi liés par covalence au niveau d'une ou plusieurs chaînes latérales des acides aminés. Dans le cas de liaison via les chaînes latérales d'acides aminés, les produits de couplage doivent conserver la propriété de se fixer avec une forte affinité sur le CMH de classe II des cellules dendritiques et d'internaliser l'antigène.

Dans les produits de couplage selon l'invention, la seconde classe de protéines est choisie dans le groupe comprenant hLAG-3, ses homologues, fragments et dérivés et les mélanges de ceux-ci.

Les homologues, fragments et dérivés de LAG-3 sont ceux capables d'assurer une fixation avec une forte affinité sur le CMH de classe II des cellules dendritiques et l'internalisation de l'antigène de la première classe de protéine.

On entend par homologue, une protéine LAG-3 d'une autre espèce que l'homme par exemple LAG-3 murin (mLAG-3). Avantageusement, la séquence protéique présente une homologie d'au moins 70 avec la séquence protéique de LAG-3 humain, de préférence d'au moins 80, et de manière particulièrement préférée d'au moins 90. L'homologie entre deux séquences protéiques correspond au pourcentage d'acides aminés identiques et localisés à une position identique ou similaire dans les deux séquences protéiques. Le pourcentage d'homologie est calculé à l'aide de l'algorithme BLAST disponible sur le site NCBI (National Center for Biotechnology Information ; http://www.ncbi.nlm.nih.go/) à l'aide de la matrice BLOSUM 62.

On entend par fragments de LAG-3, des séquences protéiques de LAG-3 capables d'assurer une fixation avec une forte affinité sur le CMH de classe II des cellules dendritiques et l'internalisation de l'antigène de la première classe de protéine, lesdites séquences protéiques présentant une longueur comprise entre 50 et 200 acides aminés, de préférence entre 60 et 175 acides aminés et de manière particulièrement préférée entre 75 et 160 acides aminés.

A titre d'exemple de fragments de hLAG-3, on peut citer tout particulièrement les fractions solubles incluant au moins les deux domaines N-terminaux extracellulaires de type Ig. Ces domaines sont décrits notamment dans les demandes de brevet internationales WO 91/10862 et WO 95/30750. L'invention envisage tout spécialement dans le produit de couplage, un fragment de LAG-3 choisi dans le groupe comprenant les fragments D1-D2 (SEQ ID No.18 et SEQ ID N.19) et D1-D4 (SEQ ID N.18, et SEQ ID N.19, SEQ ID No.20 et SEQ ID No.21). Ainsi dans les exemples rapportés dans la partie expérimentale, hLAG-3 a été utilisé sous sa forme 4 domaines Ig (D1D4) ou 2 domaines Ig (D1D2), les deux domaines D1-D2 étant suffisants pour assurer la fixation avec une forte affinité sur le CMH de classe II des cellules dendritiques puis l'internalisation.

On entend par dérivés de LAG-3 ou de ses fragments ci-dessous, ceux dont la séquence en acides aminés est modifiée par suppression, addition ou substitution d'un ou plusieurs acides aminés, lesdits dérivés étant capables d'assurer une fixation avec une forte affinité sur le CMH de classe II des cellules dendritiques et l'internalisation de l'antigène de la première classe de protéine.

On peut citer par exemple la substitution d'une ou plusieurs arginines en position 73, 75 et/ou 76 par l'acide glutamique, comme décrit dans les demandes de brevet internationales WO 95/30750 et WO 98/23741. Il peut aussi s'agir de variant d'épissage de LAG-3 comme décrit dans la demande de brevet internationale WO 98/58059.

A titre d'exemple de test de fixation susceptible de déterminer si un homologue, fragment ou dérivé de LAG-3 entre dans le cadre de l'invention, on peut citer :
- le marquage cellulaire en immunofluorescence indirecte, utilisant une lignée B transformée par EBV exprimant les molécules du CMH de classe II. On utilise toujours un contrôle positif (anticorps pan-class II appelé 9.49 ou I3 suivi d'un GAM-FITC). On est à saturation de marquage à 30 µg/ml ou 10 µg/ml de LAG-3Ig (première couche) révélé avec un GAH-FITC (deuxième couche). On a toujours la détection d'un signal à 3 ou 1 µg/ml de LAG-3Ig. Ces résultats doivent être retrouvés avec une protéine de fusion LAG-3Ig/Ag ;
- un marquage de type Biacore selon lequel on fixe une protéine MHC classe II sur une lame et on mesure l'affinité de la fixation de LAG-3Ig ou LAG-3Ig/Ag. Les deux types de "binding" doivent avoir des Kd (constante de dissociation) similaires et en tout cas inférieures à 5 x 10⁻⁸ de préférence inférieure à 3 x 10⁻⁹.

A titre d'exemple de test d'internalisation susceptible de déterminer si un homologue, fragment ou dérivé de LAG-3 entre dans le cadre de l'invention, on peut citer le marquage sur lame et analyse en microscopie confocale. Des cellules dendritiques sont obtenues en 6 jours à partir de monocytes humains purifiés (culture in vitro en présence d'IL-4 et de GM-CSF) selon la procédure suivante :
- récupérer les cellules et les compter ;
- centrifuger 5 minutes à 1100 rpm ;
- reprendre les cellules à 1 million/ml dans du PBS 1X (préalablement équilibré à 37°C) ;
- déposer 300 µl de la suspension cellulaire/lame et mettre les cellules à adhérer sur les lames recouvertes de polylysine à 37°C pendant 30 mn ;
- enlever le liquide et saturer avec 500 µl de PBS 1X / 0,1% NaN3 / 3% lait pendant 30 mn à 37°C ;
- transporter les lames au-dessus de la glace (4°C) et équilibrer la température pendant 10-15 minutes ;
- enlever le liquide et laver doucement avec du PBS 1X froid ;
- déposer 400 µl de l'anticorps (ou protéine hLAG-3Ig à 30 µg/ml) dilué dans du PBS 1X / 0,1% NaN₃ / 3% lait froid et incuber à 4°C pendant 30 mn.
- enlever le liquide et laver doucement avec du PBS 1X froid ;
- répéter ces 2 dernières étapes pour chaque marquage supplémentaire (GAH-FITC pour le marquage LAG-3Ig).
- Mettre les lames à 37°C pendant 15-20 min (dans l'incubateur pour permettre l'internalisation) ;
- déposer 400 µl de PBS 1X / 2% PFA froid et fixer les cellules pendant 15 mn à 4°C;
- enlever le liquide et laver doucement avec du PBS 1X froid ;
- déposer 20-30 µl de Fluoromount G, mettre délicatement la lamelle et laisser sécher à température ambiante pendant 1 à 2 h avant de mettre à 4°C.
- Analyser les lames en microscopie confocale.

Dans les produits de couplage selon l'invention, la première classe de protéine de type antigène est choisie dans le groupe comprenant des antigènes viraux, des antigènes bactériens, des antigènes tumoraux, des antigènes de parasites et leurs mélanges.

Ainsi, la première classe de protéine de type antigène est un antigène viral de préférence choisi dans le groupe comprenant les virus HPV, HBV, HCV, HIV, EBV, CMV et leurs mélanges, et tout spécialement, le groupe comprenant l'antigène E7 d'HPV et l'antigène gag-nef d'HIV. En effet, dans le but de développer de nouvelles protéines vaccinales, LAG-3 a été fusionné à des antigènes viraux (E7 de HPV-16 ou gag-nef de HIV-1). L'objectif est d'obtenir, en plus de l'effet adjuvant (immunostimulant) de LAG-3, un ciblage de l'antigène vers les cellules dendritiques immatures. Ces cellules expriment les molécules du CMH de classe II, ligand de LAG-3, qui sont recyclées très rapidement vers l'intérieur de la cellule entraînant ainsi l'antigène couplé à LAG-3. Des molécules de fusion entre hLAG-3Ig et les antigènes viraux ont donc été construites, exprimées dans des cellules de mammifères, purifiées et testées fonctionnellement.

La première classe de protéine de type antigène peut être aussi un antigène bactérien choisi dans le groupe des bactéries intracellulaires de la tuberculose, de la lèpre, de la listéria. Il peut encore avantageusement s'agir d'un antigène tumoral choisi dans le groupe comprenant CEA, Melan A, PSA, MAGE-3, HER2/neu, protéine E6 et E7 d'HPV (cancer du col utérin).

L'invention offre une nouvelle stratégie vaccinale basée sur l'utilisation de LAG-3 qui est basée sur l'utilisation d'un ligand naturel et pas d'un anticorps. Celle-ci offre l'avantage de permettre une diminution très forte de la dose de LAG-3Ig et d'antigène injecté, ce qui permet un développement plus facile sur un plan industriel des compositions de vaccin thérapeutique les contenant. Par ailleurs, ces compositions permettent d'induire de fortes réponses T CD8 chez l'homme avec de faibles doses de vaccins thérapeutiques. Il faut souligner l'intérêt de la démonstration de l'augmentation des réponses CD4 qui vont soutenir la réponse CD8 in vivo et permettre à cette dernière d'être très forte et prolongée c'est-à-dire efficace pour détruire des réservoirs de virus ou de cellules tumorales.

Ainsi, l'invention concerne aussi une composition de vaccin comprenant au moins un produit de couplage comme défini précédemment, avantageusement associé à un véhicule pharmaceutique sous une forme permettant l'administration *per os,* cutanée, sous-cutanée, topique, intramusculaire, intraveineuse ou intra-artérielle ou dans tous les compartiments liquidiens de l'organisme.

Avantageusement, les compositions de l'invention contiennent entre 0,1 µg/ml et 1 mg/ml,de préférence entre 0,1 µg/ml et 100 µg/ml, préférentiellement de 0,1 µg/ml et 10 µg/ml, de manière particulièrement préférée entre 0,1 µg/ml et 1 µg/ml de produit de couplage. Ces compositions sont dosées par méthode ELISA

L'invention se rapporte aussi à une méthode de vaccination d'un individu consistant à administrer à un individu atteint d'une pathologie correspondant à l'antigène de la première classe de protéine une quantité adéquate d'une composition telle que définie précédemment.

L'invention concerne encore l'utilisation d'un produit de couplage comme défini précédemment comme médicament.

Avantageusement, l'invention concerne l'utilisation d'un produit de couplage comme défini précédemment pour la préparation d'une composition immunogène capable d'induire une immunisation, de préférence capable d'induire une réponse T CD4 et/ou CD8 spécifique.

En effet, les données rapportées dans la partie expérimentale ci-après montre que le couplage de deux protéines différentes (E7 et gag-nef) en position C-terminale de la protéine LAG-3Ig permet d'obtenir *in vitro* une immunisation très forte, à la fois en ce qui concerne les réponses T CD4 (présentation par les molécules du CMH de classe II) et CD8 (présentation par-les molécules du CMH de classe I). Cette immunogénicité in vitro a été définie avec des PBMC de donneurs sains.

Les réponses CD4 ont été étudiées en Elispot par quantification des cellules sécrétant l'interféron γ intracellulaire en réponse à 48 heures à l'antigène E7 ou gag-nef présenté sous forme de protéine, qui est donc captée par les cellules dendritiques des PBMC et présenté par les molécules du CMH de classe II sous forme de peptides de 11 à 20 acides aminés.

Les réponses CD8 ont été étudiées en Elispot par quantification des cellules sécrétant l'interféron γ intracellulaire en réponse à une stimulation de 48 heures avec des peptides présentés par les molécules du CMH de classe I de 9 ou 10 acides aminés.

Dans les deux cas, une amplification préalable des réponses T CD4 ou CD8 a été obtenue après trois stimulations *in vitro* avec l'antigène.

Ces réponses fortes CD4 et CD8 en Elispot (dues au « priming » de cellules T naïves, lorsqu'il s'agit d'individus sains HPV-16⁻ et HIV⁻ ou « boosting » si les individus sains sont HPV-16⁺ ou HIV⁺) ne sont pas obtenues lorsqu'on ajoute l'antigène E7 ou gag-nef seul et à des niveaux moindres lorsqu'on ajoute un mélange LAG-3Ig et E7 ou LAG-3Ig et gag-nef.

Avantageusement encore, l'invention se rapporte également à l'utilisation d'un produit de couplage selon l'invention pour la fabrication d'un médicament destiné au traitement des maladies infectieuses et/ou du cancer. A titre d'exemple de maladies infectieuses, on peut citer les infections virales, bactériennes et parasitaires. De préférence, le traitement des maladies infectieuses ou du cancer implique une réponse immunitaire via les cellules T CD8+.

Selon un mode de réalisation particulier de l'invention, le produit de couplage selon l'invention est utilisé pour la fabrication d'un médicament destiné au traitement des maladies infectieuses et/ou du cancer dans laquelle la seconde classe de protéines de type ligand du CMH de classe II selon l'invention, est capable d'induire une réponse immunitaire antigène spécifique via les cellules T.

D'autres avantages et caractéristiques de l'invention apparaîtront des exemples qui suivent où il sera fait référence aux dessins en annexe dans lesquels :
- La figure 1 représente l'alignement de séquence nucléique (A) et peptidique (B) entre la séquence de E7wt théorique et celle de E7Rb obtenue d'après le séquençage après mutagenèse dirigée (2 mutations ponctuelles).
- La figure 2 est un résumé de la stratégie de clonage des vecteurs d'expression pCDNA3 et pSEC pour les protéines recombinantes hLAG-3Ig-E7, E7-hLAG-3Ig et le contrôle E7Rb⁻Ig. Les oligonucléotides utilisés sont représentés par les flèches
- La figure 3 représente l'alignement de séquence entre les séquences ancestrales du groupe B, consensus du groupe B et LAI, pour les protéines gag (panel A) et nef (panel B). Les acides aminés choisis pour la protéine recombinante optimisée gag-nef sont surlignés pour p17, p24 et nef.
- La figure 4 est un résumé de la stratégie de clonage des vecteurs d'expression pCDNA3 et pSEC pour les protéines recombinantes hLAG-3Ig-gagnef, gagnef-hLAG-3Ig et le contrôle gagnef-Ig. Les oligonucléotides utilisés sont représentés par les flèches
- La figure 5 représente les gels SDS-PAGE sur les protéines hLAG-3_{(D1D4)}Ig et hLAG-3_{(D1D4)}Ig-E7 purifiées; la protéine hLAG-3Ig purifiée (à 0.71 mg/ml) a aussi été déposée comme référence. Le marqueur de poids moléculaire est le même sur les trois gels (marqueur Biorad high range). A : coloration au bleu de Coomassie. B : Western blot anti-hLAG-3, avec l'anticorps monoclonal 17B4.
- La figure 6 représente la fixation de hLAG-3_{(D1D4)}Ig et hLAG-3_{(D1D4)}Ig-E7 sur le CMH de classe II de cellules B EBV-transformées (exprimée en moyenne de fluorescence pondérée par le pourcentage de cellules positives : axe des ordonnées).

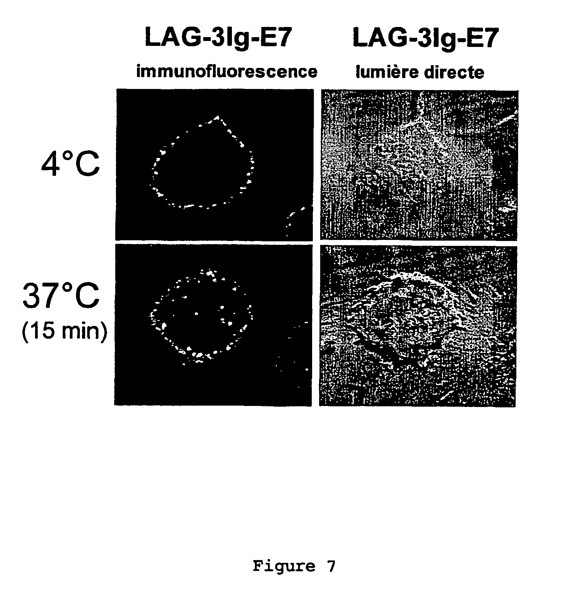

- La figure 7 représente l'internalisation à 37°C de hLAG-3_{(D1D4)}Ig-E7 dans des cellules dendritiques humaines immatures, détectée par immunofluorescence (points à l'intérieur des cellules).
- La figure 8 représente les gels SDS-PAGE sur les protéines hLAG-3_{(D1D4)}Ig/E7 et hLAG-3_{(D1D4)}Ig/gagnef purifiées; la protéine hLAG-3Ig (lot PDC012.096) a également été déposée comme référence (marqueur de poids moléculaire Kaléidoscope Biorad). A : coloration au bleu de Coomassie. B : Western blot anti-hLAG-3, avec l'anticorps monoclonal 17B4.
- La figure 9 représente la fixation de hLAG-3Ig (lot Hénogen S017/LPC/041008) , hLAG-3_{(D1D4)}Ig/E7 et hLAG-3_{(D1D4)}Ig/gagnef sur le CMH de classe II de cellules B EBV-transformées (exprimée en intensité moyenne de fluorescence en fonction de la concentration).
- La figure 10 représente l'expression des marqueurs d'activation CD40, CD80, CD83 et CD86 à la surface des cellules dendritiques incubées pendant 2 jours en présence d'IgG1 humaines, de sCD40L, de hLAG-3Ig (lot Hénogen S017/LPC/041008) , de hLAG-3_{(D1D4)}Ig/E7 ou de hLAG-3_{(D1D4)}Ig/gagnef. L'expression membranaire est proportionnelle à l'intensité de fluorescence.

### 1) Construction des vecteurs d'expression

Des vecteurs permettant l'expression et la sécrétion par des cellules de mammifères des différentes protéines recombinantes ont été construits.

### 1.1) Vecteurs utilisés

### 1.1.1) Vecteurs de clonage

Deux vecteurs d'expression ont été choisis pour l'expression des protéines recombinantes dans les cellules de hamster CHO-K1 :
- pCDNA3.1(+) de Invitrogen a été choisi pour l'expression des fusions hLAG-3Ig/antigène. Ces protéines recombinantes contiennent en N-terminal le peptide leader de hLAG-3 permettant sa sécrétion dans le milieu de culture.
- pSEC-tag2-hygroA et pSEC-tag2-hygroB de Invitrogen ont été choisis pour l'expression des fusions antigène viral/hLAG-3Ig. Ce vecteur contient un peptide leader de IgK en amont du promoteur permettant la sécrétion de la protéine.
- Dα-LAG3-DID4ΔEK-hIgG1 Fusion de Henogen a été choisi pour l'expression des fusions hLAG-3_{(D1D4)}Ig/E7 et hLAG-3_{(D1D4)}Ig/gagnef. Ce vecteur contient une séquence codant pour hLAG-3_{(D1D4)}Ig dans laquelle l'intron A, localisé en amont de la région "Ig-hinge-Fc", a été remplacée par une séquence linker (codant pour DDDDKGSGSG SEQ ID No.17) afin d'exclure des ambiguïtés d'épissage. Ce vecteur contient également le gène *dhfr* permettant la sélection des cellules ayant intégré la séquence plasmidique dans leur génome et l'amplification de cette séquence en présence de methotrexate.

### 1.1.2) Source de hLAG-3Ig

### - Vérification et amplification des plasmides de départ :

hLAG-3_{(D1D4)}Ig et hLAG-3_{(D1D2)}Ig ont été amplifiés respectivement à partir des vecteurs pCDNA3-hLAG3_{(D1D4)}-IgG1 et pCDM7-hLAG-3_{(D1D2)}IgG1.

pDNA3-hLAG3_{(D1D4)}-IgG1 : l'insert hLAG-3Ig avait été sous-cloné en XbaI dans pCDNA3 à partir de pCDM7-hLAG-3Ig.

Ces plasmides ont été ré-amplifiés à partir de stocks de bactéries transformées conservées en glycerol à -80°C. Des digestions par différentes enzymes de restrictions ont permis de confirmer la nature des plasmides

### - Séquence de hLAG-3_{(D1D4)}IgG1 et hLAG-3_{(D1D2)}IgG1 :

Les séquences de hLAG-3_{(D1D4)}IgG1 et hLAG-3_{(D1D2)}IgG1 n'étant pas connues parfaitement au commencement du projet, elles ont été entièrement séquencées après sous-clonage dans pCDNA3.1+.

Cela a permis de lever les incertitudes concernant le nombre d'introns dans IgG1 (tous les introns sont présents) et la séquence d'épissage entre hLAG-3_{(D1D2)} et IgG1 . Trois mutations dont deux changeant l'acide aminé dans la région CH3 de IgG1 ont aussi été décelées, ainsi que l'insertion d'un T en position +4 de l'intron A IgG1.

A partir de ces résultats, des séquences nommées hLAG-3_{(D1D4)}Ig reconstitué et hLAG-3_{(D1D2)}Ig reconstitué ont été compilées

### 1.2) Fusions entre hLAG-3Ig et E7 de HPV-16

Une forme mutée de E7, non oncogénique a été clonée en C-terminal ou en N-terminal de hLAG-3Ig (D1D4 ou D1D2), dans les vecteurs d'expression précédemment décrits. Une fusion entre E7 et IgGl sans LAG-3 a aussi été réalisée en guise de contrôle.

### 1.2.1) Mutagénèse de E7

Une double mutation a été réalisée dans E7 de HPV-16, de façon à empêcher sa dimérisation avec la protéine cellulaire Rb responsable du pouvoir oncogénique de E7 (voir Lee et al. Nature 1998 vol 391 p859 ; Burg et al. Vaccine 2001 vol 19 p3652 ; Boursnell et al. Vaccine vol 14 p.1485).

Un plasmide pEF6-E7, contenant la forme sauvage de E7 (clonée en T/A) a été obtenu.

Une mutagénèse dirigée dans E7 a été réalisée en utilisant le kit Quickchange de Stratagene afin de changer les acides aminés C₂₄ en G et E₂₆ en G. Le plasmide pEF6-E7 a été amplifié par PCR avec la paire d'oligonucléotides complémentaire contenant les mutations désirées :

Le produit PCR a ensuite été incubé avec Dpn1, enzyme active uniquement sur les sites méthylés, digérant donc l'ADN matrice sur les produits PCR. Le produit obtenu à ensuite été transformé dans des bactéries XL1-Blue.

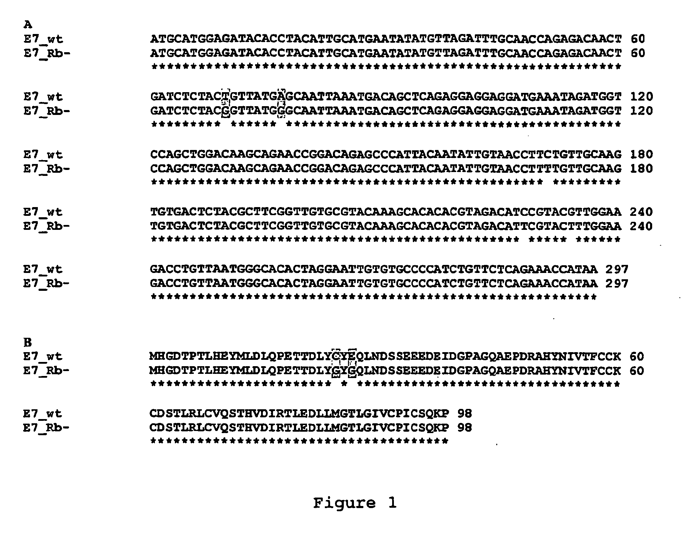

Le plasmide ainsi obtenu a été vérifié par restriction et par séquençage de l'insert E7mutRb-. Le résultat de la séquence indique que les mutations désirées sont bien présentes. Trois autres mutations n'affectant pas la composition en acides aminés ont aussi été détectées par rapport à la séquence théorique de E7 indiqué à la figure 1.

### 1.2.2) Clonage hLAG-3Ig-E7 et E7-hLAG-3Ig dans les vecteurs d'expression

Les inserts E7/Rb- (nommés désormais E7 pour simplification) et hLAG-3Ig ont été amplifiés par PCR, avec des oligonucléotides permettant l'addition d'un site de restriction aux extrémités.

L'enzyme haute fidélité Pfu turbo de Stratagene a été utilisée pour les PCR.

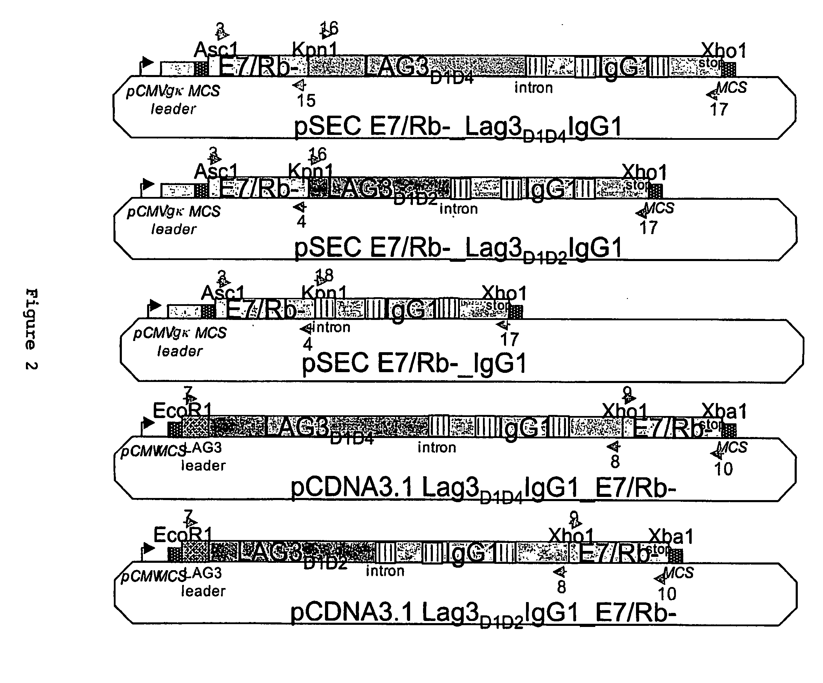

La stratégie de clonage est résumée sur la figure 2.

### - Clonage de hLAG-3Ig-E7 dans pCDNA3.1 :

E7 a été amplifié à partir de pEF6_E7/Rb- avec la paire d'oligonucléotides suivants :
oligo 9 (E7 5'-Xho1) :
CCGCTCGAGATGCATGGAGATACACCTAC (SEQ ID No.3)
contenant un site Xho1 et l'ATG de E7
oligo 10 (E7 3'-stopXba1) :
GCTCTAGATTATGGTTTCTGAGAACAG (SEQ ID No.4)
contenant la partie 3' de E7 avec le stop et un site XbaI.

Le produit PCR a été digéré par XhoI et XbaI avant purification.

LAG-3_{(D1D2)}IgG1 et LAG-3_{(D1D4)}IgG1 ont été amplifiés à partir de pCDM7-LAG-3_{D1D2}_*-IgG1 et pCDNA3-LAG-3_{D1D4}-IgG1 respectivement avec la paire d'oligonucléotides suivant :
oligo 7 (Lag3 5'-atgECoRI) :
GGAATTCGCCCAGACCATAGGAGAGATG (SEQ ID No.5)
contenant un site EcoRI, l'ATG de hLAG-3, avec le signal peptide pour la sécrétion,
oligo 8 (IgG1 3'-XhoI):
CCGCTCGAGTTTACCCGGGGACAGGGAG (SEQ ID No.6)
contenant la partie 3' de IgGI, sans le stop.

Le produit PCR a été digéré par EcoRI et XhoI avant purification.

L'insertion de hLAG-3_{(D1D4)}Ig-E7 dans pCDNA3.1+ a été réalisée en deux étapes. Premièrement, hLAG-3_{(D1D4)}Ig a été ligaturé dans pCDNA3.1+ digéré par EcoRI et XhoI. On obtient ainsi l'intermédiaire de clonage pCDNA-hLAG-3_{(D1D4)}Ig. Puis E7 a été inséré dans pCDNA-hLAG-3_{(D1D4)}Ig préalablement digéré par XhoI et XbaI.

Pour le clonage de la fusion hLAG-3_{(D1D2})Ig-E7 dans pCDNA3.1+, les deux inserts ont en premier lieu été ligaturés ensemble. Le fragment hLAG-3_{(D1D2})Ig-E7 ainsi obtenu a été purifié sur gel puis inséré directement dans pCDNA3.1+ préalablement digéré par EcoRI et XbaI.

Un plasmide pCDNA-hLAG-3_{(D1D2)}Ig a également été fabriqué en ligaturant hLAG-3_{(D1D2)}Ig dans pCDNA3.1+ digéré par EcoRI et XhoI.

Les inserts de ces vecteurs d'expression ont été séquencés. Les résultats des séquences confirment la bonne insertion en phase des inserts

### - Clonage de E7-hLAG-3Ig dans pSECtag-hygroA :

E7 a été amplifié à partir de pEF6-E7/Rb- avec la paire d'oligonucléotides suivant :
oligo 3 (E7 5'-AscI) :
GGCGCGCCATGCATGGAGATACACCTAC (SEQ ID No.7)
contenant un site AscI et l'ATG de E7,
oligo 15 (E7 3'-Kpnl) :
GGGGTACCTGGTTTCTGAGAACAGATG (SEQ ID No.8)
contenant la partie 3' de E7 sans stop et un site KpnI.

Le produit PCR a été digéré par AscI et KpnI avant purification.

LAG-3_{(D1D2)}IgG1 et LAG-3_{(D1D4)}IgG1 ont été amplifiés à partir de pCDM7-LAG-3_{D1D2}-IgG1 et pCDNA3-LAG-3_{D1D4}-IgG1 respectivement avec la paire d'oligonucléotides suivant :
oligo 16 (Lag3 5'-D1KpnI) :
GGGGTACCCTCCAGCCAGGGGCTGAG (SEQ ID No.9)
contenant un site KpnI et la partie 5' du domaine 1, sans le signal peptide,
oligo 17 (IgGI 3'-stopXho1) :
CCGCTCGAGTCATTTACCCGGGGACAG (SEQ ID No.10)
contenant la partie 3' de IgGI avec le stop et un site XhoI.

Le produit PCR a été digéré par KpnI et XhoI avant purification.

IgGI a été amplifié à partir de pCDNA3-LAG-3_{D1D4}-IgG1 (pour le clonage dans pSEC en 3' de E7 pour utilisation comme contrôle sans Lag3) avec la paire d'oligonucléotides suivant :
oligo 18 (IgG1 5'Kpn1) :
GGGGTACCCGAGGGTGAGTACTAAGC (SEQ ID No.11)
contenant un site KpnI et la partie 5' de l'intron A de IgGI,
oligo 17(IgGI 3'-stopXhoI) :
CCGCTCGAGTCATTTACCCGGGGACAG (SEQ ID No.12)
contenant la partie 3' de IgGI avec le stop et un site XhoI.

Le produit PCR a été digéré par KpnI et XhoI avant purification

L'insertion des fusions E7-hLAG-3Ig dans pSECtag-hygroA a été réalisée en deux étapes :
- le produit PCR de E7 digéré a été ligué dans pSECB préalablement digéré par AscI et KpnI. On obtient ainsi un intermédiaire de clonage nommé pSEC-E7 ;
- les produits PCR de hLAG-3_{(D1D4)}Ig, hLAG-3_{(D1D2)}Ig et IgGI ont ensuite été ligués dans pSEC-E7 préalablement digéré par KpnI et XhoI.

Ces vecteurs d'expression ont été vérifiés par restriction enzymatique.

### -Clonage de E7 dans Dα-LAG3-D1D4-ΔEK-hIgG1 Fusion :

Le vecteur de destination est le vecteur Dα-LAG3-DID4ΔEK-hIgG1 Fusion de Henogen. Dans ce vecteur, la séquence codant pour LAG-3_{(DID4)}Ig est insérée entre les deux sites de restriction XhoI. Comme mentionné au-dessus, ce vecteur contient la séquence codant pour LAG-3_{(DID4)}Ig dans laquelle l'intron A (intron en position 5' de la région "Hinge" de Ig) a été remplacé par une séquence linker (codant pour DDDDKGSGSG : SEQ ID No.17). Cette séquence codant pour LAG-3_{(DID4)}Ig dépourvu d'intron A sera notée LAG-3_{(DID4)}Ig/ et par extension la même notation sera conservée pour la protéine codée par ces constructions.

Le fragment codant pour E7 est issu du plasmide pcDNA3-LAG3_{(DID4)}-E7.

Le clonage de E7 dans Dα-LAG3-D1D4-ΔEK-hIgG1 Fusion a été effectué en deux étapes :

Dans une première étape, le fragment LAG-3_{(DID4)}-Ig/ a été inséré dans pCDNA3-LAG-3_{(DID4)}Ig-E7.

Le vecteur Dα-LAG3-D1D4-ΔEK-hIgG1 a été clivé par XhoI et les extrémités cohésives rendues franches par l'enzyme T4 polymérase. Le fragment correspondant au vecteur Dα dépourvu de l'insert XhoI blunt/XhoI blunt a été conservé comme vecteur de destination finale. L'insert XhoI blunt/XhoI blunt a été digéré par l'enzyme BsrGI au niveau de l'intron C de la séquence codant pour Ig afin d'éliminer les 300 dernières paires de bases de la séquence codant pour Ig qui inclut le codon Stop (insert LAG-3_{(DID4)}Ig/ XhoI blunt/BsrGI).

Le vecteur pCDNA3-LAG-3_{(DID4)}Ig-E7 a été digéré par l'enzyme EcoRI (site EcoRI contenu dans le Multiple Cloning Site de pCDNA3 en amont du site de clonage) et les extrémités cohésives rendues franches par l'enzyme T4 polymérase. Le plasmide ainsi linéarisé a été clivé par l'enzyme BsrGI afin d'éliminer la séquence codant pour LAG-3_{(DID4)} et la séquence 5' codant pour Ig afin de conserver les 300 dernières paires de base codant pour Ig (pcDNA EcoRI blunt/BsrGI Ig-E7).

L'insert LAG-3_{(DID4)}Ig/ XhoI blunt/BsrGI a été ligaturé dans pCDNA3 EcoRI blunt/BsrGI Ig-E7.

Après analyse par restriction des clones obtenus, un clone contenant pCDNA3-LAG-3_{(DID4)}Ig/E7 a été sélectionné.

Dans une seconde étape, la construction LAG-3_{(DID4)}Ig/E7 a été cloné dans Dα.

L'insert LAG-3_{(DID4)}Ig/E7 contenu dans pCDNA3 a été clivé par l'enzyme PmeI (2 sites PmeI encadrant le Multiple Cloning Site de pCNDA3, bout franc) et ligaturés dans Dα sans insert préalablement préparé par clivage XhoI blunt/XhoI blunt et déphosphorylé.

Les clones possédant l'insert LAG-3_{(DID4)}Ig/E7 dans Dα dans la bonne orientation ont été sélectionnés après analyse par restriction.

L'ADN d'un des clones Da-LAG-3_{(DID4)}Ig/E7 a été retransformé dans la souche DH5a et préparé par maxiprep Endofree (Qiagen) et utilisé pour la transfection transitoire dans des cellules CHO-K1 afin de vérifier le produit de traduction du plasmide. Le vecteur d'origine Dα-LAG3-D1D4-ΔEK-hIgG1 Fusion et pCDNA3-LAG-3_{(DID4)}Ig ont été utilisés comme contrôles positifs et le vecteur Dα- dont l'insert a été éliminé par XhoI, suivie d'une ligaturation comme contrôle négatif. Vingt-quatre heures après la transfection, les surnageants ont été quantifiés en LAG-3 par un test ELISA spécifique. Parallèlement, les protéines recombinantes présentes dans les surnageants et les lysats cellulaires ont été précipitées par protéine A-sepharose (Pharmacia) et analysées par western blot anti-LAG3 pour estimer leur taille. Les poids moléculaires apparents étaient ceux attendus.

L'ADN du clone Da-LAG-3_{(DID4)}Ig/E7 sélectionné est celui utilisé pour la transfection stable dans les cellules CHO-*dhfr⁻.*

### 1.3) Fusions entre hLAG-3Ig et gag-nef de HIV-1

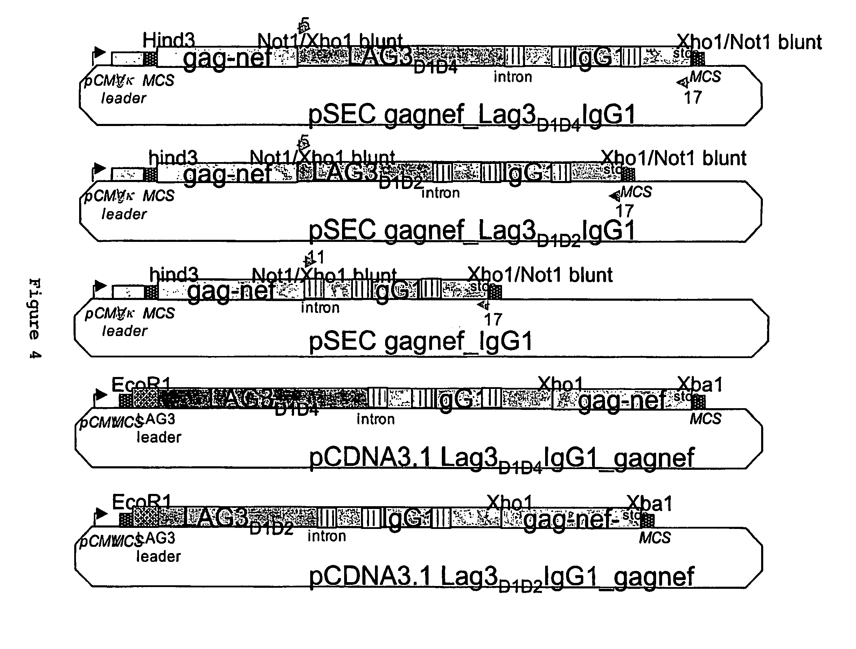

Cet antigène de HIV-1 a été fusionné à hLAG-3Ig (D1D4 ou D1D2), soit en C-terminal, soit en N-terminal, et cloné dans les vecteurs d'expression précédemment décrits. Une fusion entre cet antigène et IgG1 sans LAG-3 a aussi été réalisée en guise de contrôle (figure 4).

L'antigène de HIV-1 choisi pour faire cette protéine recombinante vaccinale est une fusion optimisée entre gag p17, gag p24 et une partie de nef.

### 1.3.1) Séquence gag-nef utilisée

La séquence de la protéine chimère entre gag p17, gag p24 et nef a été définie de façon à avoir un maximum de chance d'être retrouvée chez les patients européens (souches B).

Pour cela, nous avons comparé pour chaque protéine des séquences peptidiques suivantes obtenues sur le site Web http://hiv-web.lanl.gov/content/hiv-db/CONSENSUS/M GROUP/ 2002-Aug.html.

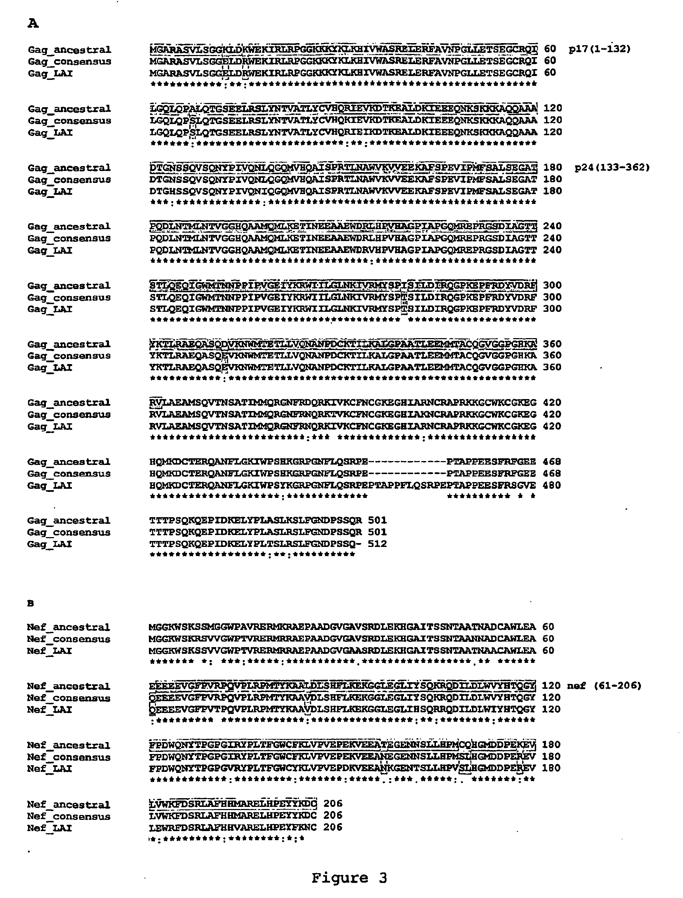

L'alignement des séquences suivantes est représenté à la figure 3 :
- La séquence ancestrale de la souche B (séquence théorique de laquelle les virus actuels du groupe B dérivent, a été réalisée à partir de l'arbre phylogénétique).
- La séquence consensus de la souche B (consensus entre toutes les séquences actuelles du groupe B, ne prenant pas en compte leur représentation).
- La séquence de la souche LAI (1^{er} isolat européen).

La séquence retenue pour chaque protéine p24, p17 et nef correspond à la séquence ancestrale, sauf quand un acide aminé diffère à la fois de LAI et de consensus, qui elles sont identiques pour cet acide aminé. Nous considérons dans ce cas qu'il y a plus de chance de retrouver dans la population actuelle la séquence consensus et LAI que l'ancestral (figure 3). Ces trois séquences peptidiques ont ensuite été mises bout à bout. Les 60 premiers acides aminés de nef ont été supprimés car ils ne contiennent pas d'épitope T majeurs détectés chez les patients et sont responsables de l'effet cytopathogène de nef.

La séquence ADN correspondant à la séquence peptidique ainsi obtenue pour la chimère gag-nef a été optimisée pour l'expression dans les cellules de hamster (cellules CHO-K1) par la société ATG-Biosynthétics. Des sites de restriction ont été ajoutés en 5' et en 3' de gagnef pour permettre le sous-clonage. Le gène a ensuite été synthétisé par la société ATG-Biosynthetics et livré dans le vecteur pCR4topo.

### 1.3.2) Clonage de hLAG-3Ig-gagnef et gagnef-hLAG-3Ig dans les vecteurs d'expression

La stratégie de clonage est résumée sur la figure 4.

### - Clonage de hLAG-31g-gagnef dans pCDNA3.1 :

Gag-nef a été sous-cloné à partir de pCR4topo-gagnef dans les vecteurs pCDNA3.1-hLAG3_{(DID4)}Ig et pCDNA3.1-hLAG3_{(D1D2)}Ig en XhoI et XbaI.

Les vecteurs d'expression pCDNA-hLAG3_{(D1D4)}Ig-gagnef et pCDNA-hLAG3_{(D1D2)}Ig-gagnef ont été vérifiés par digestions enzymatiques

### - Clonage de gagnef-hLAG-31g dans pSECtag-hybroB :

Ce clonage a été réalisé en trois étapes :
- Première étape : sous-clonage de gag-nef à partir de pCR4topo-gagnef dans pSECtag-hygroB en Hind3 et Not1. On obtient ainsi un intermédiaire de clonage pSEC-gagnef.
- Deuxième étape : clonage de hLAG-3Ig (D1D4 et D1D2) dans pSECtaghygroB en XhoI. Cette étape constitue un intermédiaire de clonage en vue d'obtenir de grandes quantités d'insert digéré par XhoI. En effet, si les produits PCR avaient été directement digérés par XhoI, un grand nombre de fragments non digérés auraient ensuite été clonés en « blunt » entraînant des faux positifs impossibles à éliminer autrement que par séquençage.

hLAG-3_{(D1D4)}Ig et hLAG-3_{(D1D2)}Ig ont été amplifiés par PCR à partir de pCDNA3-Lag3_{D1D4}-IgGI et pCDM7-Lag3_{D1D2}-IgG1 respectivement avec la paire d'oligonucléotides suivant :
oligo 5 (Lag3 5'-D1XhoI) :
CCGCTCGAGCTCCAGCCAGGGGCTGAG (SEQ ID No.13)
contenant un site XhoI et la partie 5' du domaine 1, sans le signal peptide.
oligo 17 (IgGI 3'-stopXhoI) :
CCGCTCGAGTCATTTACCCGGGGACAG (SEQ ID No.14)
contenant la partie 3' de IgGI avec le stop et un site XhoI
IgGI a été amplifié par PCR à partir de pCDNA3-Lag3_{D1D4}-IgG1 avec la paire d'oligonucléotides suivante :
oligo 11(IgG1 5'XhoI) :
CCGCTCGAGCGAGGGTGAGTACTAAGC (SEQ ID No.15)
contenant un site XhoI et la partie 5' de l'intron A de IgGI.
oligo 17 (IgG1 3'-stopXhoI) :
CCGCTCGAGTCATTTACCCGGGGACAG (SEQ ID No.16)
contenant la partie 3' de IgGI avec le stop et un site XhoI.

Les produits PCR ont été digérés par XhoI avant purification. Ils ont ensuite été ligaturés dans pSECtag-hygroB préalablement digéré par XhoI
- Troisième étape : clonage de hLAG-3_{(D1D4)}Ig et hLAG-3(_{D1D2})Ig dans pSEC-gagnef.

Les inserts hLAG-3_{(D1D4)}Ig, hLAG-3_{(D1D2})Ig et IgGI ont été ressortis de pSECtag-hybroB par digestion avec XhoI, les extrémités 5' sortantes ont été rendues franches par la PNK. Le vecteur pSEC-gagnef a été digéré par NotI, les extrémités cohésives ont aussi été rendues franches par la PNK. Inserts et vecteurs purifiés ont été ligaturés.

Les jonctions entre les inserts de ces vecteurs d'expression ont été séquencées pour vérifier que le cadre de lecture a été respecté.

### -Clonage de gagnef dans Dα-LAG3-D1D4-ΔEK-hIgG1 Fusion :

Le vecteur de destination est celui utilisé par Henogen pour la production de LAG-3_{(DID4)}Ig, Dα-LAG3-D1D4-ΔEK-hIgGl Fusion.

Le fragment codant gagnef est issu de pCDNA3-LAG3_{(D1D4})Ig-gagnef.

Le clonage de gagnef dans Dα-LAG3-D1D4-ΔEK-hIgG1 Fusion a été effectué en deux étapes :

Dans une première étape, le fragment LAG-3_{(DID4)}-Ig/ a été inséré dans pCDNA3-LAG-3_{(DID4)}Ig-gagnef.

Le vecteur Dα-LAG3-D1D4-ΔEK-hIgG1 a été clivé par l'enzyme XhoI et les extrémités cohésives rendues franches par l'enzyme T4 polymérase. Le fragment correspondant au vecteur Dα sans insert XhoI blunt/XhoI blunt a été conservé comme vecteur de destination finale. L'insert XhoI blunt/XhoI blunt a été digéré par l'enzyme BsrGI clivant dans l'intron C de la séquence codant pour Ig afin d'éliminer les 300 dernières paires de bases de la séquence codant pour Ig incluant le codon Stop (insert LAG-3_{(DID4)}Ig/ XhoI blunt/BsrGI).

Le vecteur pCDNA3-LAG-3_{(DID4)}Ig-gagnef a été digéré par l'enzyme EcoRI (site EcoRI contenu dans le Multiple Cloning Site de pCDNA3 en amont du site de clonage) et les extrémités cohésives rendues franches par l'enzyme T4 polymérase. Le plasmide ainsi linéarisé a été clivé par l'enzyme BsrG1 pour éliminer la séquence codant pour LAG-3_{(DID4}) et la séquence 5' codant pour Ig et conserver les 300 dernières paires de base de la séquence codant pour Ig (pCDNA EcoRI blunt/BsrGI Ig-gagnef). Le fragment LAG-3_{(DID4)}Ig/ XhoI blunt/BsrGI a été ligaturé dans pcDNA3 EcoRI blunt/BsrGI Ig-gagnef.

Après analyse par restriction des clones obtenus, un clone contenant pCDNA3-LAG-3_{(DID4})Ig/gagnef a été sélectionné.

Dans une seconde étape, LAG-3_{(DID4)}Ig/gagnef a été cloné dans Dα

La totalité de l'insert LAG-3_{(D1D4)}Ig/gagnef contenu dans pCDNA3 a été sorti par digestion avec PmeI (2 sites encadrant le Multiple Cloning Site de pCDNA3, bout franc) et cloné dans Dα sans insert préalablement préparé par clivage XhoIblunt/XhoIblunt et déphosphorylé.

Les clones possédant l'insert LAG-3_{(D1D4)}Ig/gagnef dans Dα dans la bonne orientation ont été sélectionnés après analyse par restriction.

L'ADN d'un des clones Dα-LAG-3_{(DID4)}-Ig/gagnef a été retransformé dans la souche DH5α et préparé par maxiprep Endofree (Qiagen) et utilisé pour la transfection transitoire dans des cellules CHO-K1 afin de vérifier le produit de traduction du plasmide. Le vecteur d'origine Dα-LAG3-D1D4-ΔEK-hIgG1 Fusion et pCDNA3-LAG-3_{(DID4)}Ig ont été utilisés comme contrôles positifs et le vecteur Dα - dont l'insert a été éliminé par XhoI, suivi d'une religaturation - comme contrôle négatif. Vingt-quatre heures post-transfection, les surnageants ont été quantifiés en LAG-3 par un test ELISA spécifique. Parallèlement, les protéines recombinantes présentes dans les surnageants et les lysats cellulaires ont été précipitées par protéine A-sepharose (Pharmacia) et analysées par western blot anti-LAG3 pour estimer leur taille. Les poids moléculaires apparents étaient ceux attendus.

L'ADN du clone Dα-LAG-3_{(DID4)}Ig/gagnef sélectionné est celui utilisé pour la transfection stable dans les cellules CHO-*dhfr*⁻.

### 2) Etablissement de lignées stables de cellules CHO exprimant les protéines de fusion

### 2.1) Etablissement de lignées stables exprimant les protéines de fusion à partir des vecteurs pCDNA3 et pSEC.

Les vecteurs d'expression construits précédemment en pCDNA3 ou pSEC ont été transfectés dans des cellules CHO-K1 en vue d'obtenir des lignées productrices stables exprimant les protéines recombinantes d'intérêt.

### 2.1.1) Méthode de transfection et d'isolement des clones

### 2.1.1.1) Digestion des plasmides

Pour maximiser les chances d'obtenir des transfectants stables exprimant la protéine d'intérêt, les vecteurs d'expression ont été linéarisés avant transfection. L'enzyme de restriction choisie est Bgl2, qui digère en position 1 les vecteurs pCDNA et pSEC, mais aucun des inserts hLAG-3Ig, gagnef ou E7. Les digestions ont été faites sur 10 µg de vecteur avec 30U d'enzyme, l'ADN a ensuite été précipité et repris dans 20 µl de H2O UP.

Toutefois, une étude comparative a permis de déterminer que cette étape de linéarisation n'est pas cruciale puisque le nombre de transfectants stables positifs obtenu avec un plasmide linéarisé ou non diffère peu.

### 2.1.1.2) Transfections

Les transfections ont toutes été faites entre 3 et 4 fois de façon indépendante dans des cellules CHO-K1 (réf. ECCAC n°85051005) ayant un nombre de passages compris entre 8 et 14.

Brièvement, les cellules CHO-K1 à semi confluence en plaques 6 puits sont transfectées avec 2 µg de plasmide, en utilisant 5 µl de Lipofectamine (GIBCO).

### 2.1.1.3) Sélection de clones résistants

24 heures après transfection, les cellules ont été trypsinées et ensemencées dans une boîte ronde de 150 mm, en présence de milieu contenant l'antibiotique de sélection. L'antibiotique de sélection est le G418 à 0.5 mg/ml pour les vecteurs pCDNA, et l'hygromycine B à 0,4 mg/ml pour les vecteurs pSEC. Le milieu a été changé tous les deux à trois jours jusqu'à l'apparition de clones isolés de cellules (1 à 2 semaines).

Les clones de cellules résistantes à l'antibiotique, ayant donc intégré le plasmide, ont été repiqués manuellement sous le microscope avec une pipette P200, et transférés en plaques 96 puits. Chaque clone a ensuite été testé pour l'expression de la protéine d'intérêt, les clones positifs ont alors été amplifiés.

### 2.1.1.4) Dénomination des clones

Les clones ont été nommés selon un code permettant d'identifier la date de la transfection de laquelle ils sont issus et le plasmide qu'ils contiennent.

Les deux premiers chiffres correspondent au jour et au mois de la transfection, suivi par une lettre correspondant au plasmide transfecté, puis le numéro du clone. Par exemple, un clone obtenu à partir de la transfection du 7 Janvier, avec le plasmide pCDNA sera nommé 07_01_Ax (x étant un numéro de clone).

A chaque plasmide a été attribuée une lettre selon la nomenclature suivante :
A : pCDNA
B : pCDNA-hLAG-3_{(D1D4)}Ig
C : pCDNA-hLAG-3_{(D1D2)}Ig
D : pCDNA-hLAG-3_{(D1D4)}Ig-E7
E : pCDNA-hLAG-3_{(D1D2)}Ig-E7
F : pSEC
G : pSEC-E7- hLAG-3_{(D1D4)}Ig
H : pSEC-E7- hLAG-3_{(D1D2)}Ig
I : pSEC-E7-IgG1
J : pCDNA-hLAG-3_{(D1D4)}Ig-gagnef
K : pCDNA-hLAG-3_{(D1D2)}Ig- gagnef
L : pSEC- gagnef - hLAG-3_{(D1D4)}Ig
M : pSEC- gagnef - hLAG-3_{(D1D2)}Ig
N : pSEC- gagnef -IgGl

### 2.1.2) Sélection des meilleurs clones producteurs des protéines recombinantes hLAG-3Ig-E7

La première série de plasmides transfectés ont été pCDNA-LAG-3_{(D1D4)}Ig-E7, pCDNA-LAG-3_{(D1D2)}Ig-E7, ainsi que pCDNA-LAG-3_{(D1D4)}Ig et pCDNA-LAG-3(_{D1D2)}Ig, comme contrôle sans E7.

### 2.1.2.1) Analyse de l'efficacité de transfection transitoire

L'efficacité de transfection des cellules a tout d'abord été évaluée par analyse au FACS après marquage intracellulaire avec un anticorps anti-LAG3 (17B4), 24h après transfection. Il s'avère que l'efficacité de transfection transitoire est bonne pour les plasmides pCDNA-hLAG-3_{(D1D4)}Ig, pCDNA-hLAG-3_{(D1D2)}Ig et pCDNA-hLAG-3_{(D1D2)}Ig-E7 (entre 40 et 50% de cellules positives) mais beaucoup plus faible pour pCDNA-hLAG-3_{(D1D4})Ig-E7.

### 2.1.2.2) Test des clones de transfectants stables

Cette série de transfections a été réalisée 4 fois : le 07/01/2003, le 14/01/2003, le 21/01/2003 et le 27/03/2003.

Les surnageants de chaque clone de cellule transfectée ont été testés en ELISA anti-LAG-3, purs ou dilués 2 fois selon les cas.

Les meilleurs clones de chaque série de transfection ont été amplifiés, deux ampoules par clone ont été congelées. Ces clones ont ensuite été comparés les uns aux autres par analyse ELISA de leurs surnageants pour ne garder que les meilleurs clones producteurs.

Clones congelés et comparés ensuite les uns par rapport aux autres :
pour pCDNA : 7-1-A1, 14-1-A2, 21-1-A2,
pour pCDNA-hLAG-3_{(D1D4)}Ig : 7-1-B1, 7-1-B3, 7-1-B4, 14-1-B8 ,14-1-B14, 21-1-B11, 27-6-B6
pour pCDNA-hLAG-3_{(D1D2)}Ig : 7-1-Cl, 7-1-C2, 14-1-C12, 14-1-C16, 21-1-C8, 27-5-C5
pour pCDNA-hLAG-3_{(D1D4)}Ig-E7 : 7-1-D3, 14-1-D4, 14-1-D8, 21-1-D1, 27-5-D3
pour pCDNA-hLAG-3_{(D1D2)}Ig-E7 : 7-1-E1, 7-1-E3, 7-1-E5, 14-1-E9, 14-1-E11, 21-1-E6, 27-5-E8.

Les meilleurs clones ont aussi été comparés par analyse de leurs surnageants en Western blot. Tout cela a permis de choisir un clone pour chaque plasmide transfecté qui sera amplifié et servira de cellules productrices des protéines recombinantes.

Les clones choisis comme lignées productrices des protéines recombinantes sont :
pour pCDNA : 7-1-A1
pour pCDNA-hLAG-3_{(D1D4)}Ig : 7-1-B4
pour pCDNA-hLAG-3_{(D1D2)}Ig : 21-1-C8
pour pCDNA-hLAG-3_{(D1D4)}Ig-E7 : 7-1-D3
pour pCDNA-hLAG-3_{(D1D2)}Ig-E7 : 14-1-E11

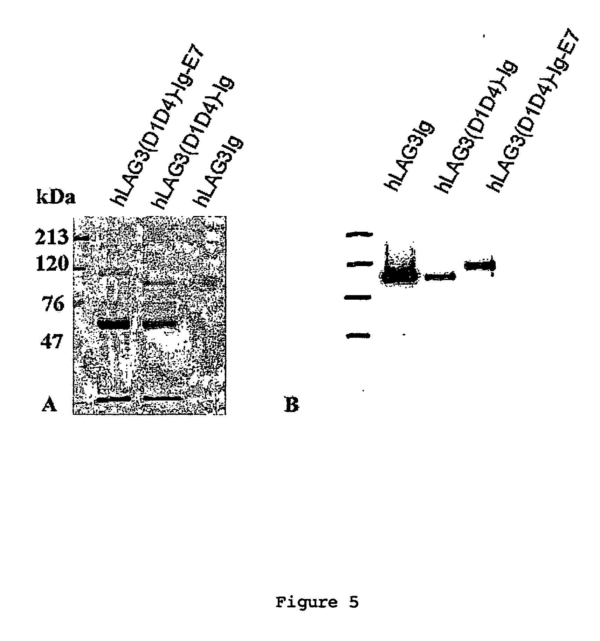

hLAG-3_{(D1D4)}Ig-E7 migre comme attendu un peu plus haut que hLAG-3_{(D1D4)}Ig (figure 5). Les deux bandes observées correspondent aux formes monomériques et dimériques de hLAG-3Ig, la réduction par chauffage en présence de β-mercaptoéthanol n'ayant pas été suffisamment efficace.

### 2.1.2.3) Congélation des banquets de cellules productrices

Une des deux ampoules de secours ayant été congelée à faible passage a été décongelé puis réamplifiée jusqu'à obtenir une flasque de 175 cm2 à confluence à partir de laquelle 5 ampoules ont été congelées comme "Master Cell Bank" le 03/03/2003. Le nombre de passages depuis l'isolement des clones est alors compris entre 5 et 7 selon les lignées.

Cinq autres ampoules ont ensuite été congelées à partir d'une flasque de 175 cm2 comme « Working Cell Bank » le 05/03/2003.)

### 2.1.3) Sélection des meilleurs clones producteurs des protéines recombinantes E7-hLAG-3Ig

### 2.1.3.1) Analyse de l'efficacité de transfection transitoire

L'efficacité de transfection des cellules a été évaluée par analyse au FACS après marquage intracellulaire avec un anticorps anti-human IgG couplé au FITC, 24h après transfection. L'efficacité de transfection transitoire est meilleure pour pSEC-E7-hLAG-3_{(D1D4)}Ig que pour pSEC-E7-hLAG-3_{(D1D2)}Ig ou pSEC-E7-IgGl. Bien que la qualité de préparation de l'ADN soit meilleur (kit genelute SIGMA remplacé par kit midiprep Qiagen), l'efficacité de transfection transitoire des plasmides pSEC est inférieure à celle obtenue avec les plasmides pCDNA (moins de 10% contre 50%).

### 2.1.3.2) Test des clones de transfectants stables

Cette série de transfections a été réalisée quatre fois : le 23/04/03, le 07/05/2003, le 19/06/2003 et le 27/06/2003.

Les surnageants de tous les clones de cellules transfectées par pSEC-E7-hLAG-3_{(D1D4)}Ig et pSEC-E7-hLAG-3_{(D1D2)}Ig ont été testés purs en ELISA anti-LAG-3. Les DO des surnageants de cellules exprimant E7-LAG3 sont beaucoup plus faibles que celles de cellules exprimant LAG3-E7.

Les clones de cellules transfectées par pSEC-E7-IgG1 exprimant une fusion ne contenant pas LAG3 ne peuvent évidemment pas être analysés par ELISA. Ils ont tous été testés par analyse au FACS après marquage intracellulaire avec un anticorps anti-IgG humaine couplée au FITC.

Les meilleurs clones de chaque série de transfection ont été amplifiés, deux ampoules par clone ont été congelées. Clones congelés et comparés ensuite les uns aux autres :
pour pSEC : 23-04-F1, 07-05-F1
pour pSEC-E7-hLAG-3_{(D1D4})Ig : 23-04-G3, 07-05-G27, 07-05-G32, 19-06-G8, 19-06-G40
pour pSEC-E7-hLAG-3_{(D1D2})Ig : 23-04-H10, 07-05-H11
pour pSEC-E7-IgG1 : 23-04-I12, 07-05-I13, 19-06-I4, 19-06I19.

Ces clones ont ensuite été comparés les uns aux autres par analyse ELISA de leurs surnageants, ou par marquage intracellulaire, pour ne garder que les meilleurs clones producteurs. Les clones choisis comme lignée productrice des protéines recombinantes sont :
pour pSEC : 07-05-F1 ;
pour pSEC-E7-hLAG-3_{(D1D4)}Ig : 19-06-G8 ;
pour pSEC-E7-hLAG-3_{(D1D2)}Ig : aucun clone n'a été amplifié, car la DO des surnageants était faible par rapport aux clones E7-D1D4. Les deux ampoules de 07-05-H11 ont toutefois été conservées dans l'azote liquide ;
pour pSEC-E7-IgGl : 07-05-I13.

### 2.1.3.3) Congélation des banques de cellules productrices

Cinq ampoules des lignées 19-06-G8 et 07-05-I13 ont été congelées à partir d'une flasque de 175 cm2 à confluence comme "Master Cell Bank" le 17/05/2003. le nombre de passages depuis l'isolement des clones est alors compris entre 5 et 8 selon la lignée.

Cinq autres ampoules ont ensuite été congelées à partir d'une flasque de 175 cm2 comme "Working Cell Bank" le 21/07/2003.

### 2.1.4) Sélection des meilleurs clones producteurs des protéines recombinantes hLAG-3Ig-gagnef

### 2.1.4.1) Analyse de l'efficacité de transfection transitoire

L'efficacité de transfection a été évaluée par analyse au FACS après marquage intracellulaire avec un anticorps anti-human IgG couplé au FITC sur des cellules transfectées transitoirement. L'efficacité de transfection transitoire est d'environ 10%.

### 2.1.4.2) Test des clones de transfectants stables

Cette série de transfections a été réalisée 3 fois : le 07/05/2003, le 19/06/2003 et le 27/06/2003.

Les surnageants de chaque clone de cellule transfectée ont été testés purs en ELISA anti-LAG-3.

Les meilleurs clones de chaque série de transfection ont été amplifiés, deux ampoules par clone ont été congelées. Clones congelés et comparés ensuite les uns aux autres :
pour pCDNA-hLAG-3_{(D1D4)}Ig-gagnef : 07-05-J2, 07-05-J23, 07-05-J53, 07-05-J71, 19-06-J18, 19-06-J32, 19-06-J48
pour pCDNA-hLAG-3_{(D1D2)}Ig-gagnef : 07-05-K19, 07-05-K71, 19-06-K33, 19-06-K43, 19-06-K44, 27-06-K7

Ces clones ont ensuite été comparés les uns aux autres par analyse ELISA de leurs surnageants, ou par marquage intracellulaire, pour ne garder que les meilleurs clones producteurs. Les clones choisis comme lignées productrices des protéines recombinantes sont :
pour pCDNA-hLAG-3_{(D1D4)}Ig-gagnef : 07-05-J53 ;
pour pCDNA-hLAG-3_{(D1D2)}Ig-gagnef : 07-05-K19 et 27-06-K7.

### 2.1.4.3) congélation des banquets de cellules productrices

Cinq ampoules des lignées 07-05-J53 et 07-05-K19 ont été congelées à partir d'une flasque de 175cm2, ainsi que 3 ampoules de 27-06-K7 à partir d'une flasque de 185cm2, comme "Master Cell Bank" le 17/05/2003. le nombre de passages depuis l'isolement des clones est alors compris entre 4 et 8 selon la lignée.

Cinq ou trois autres ampoules ont de même été congelées comme "Working Cell Bank" le 21/07/2003.

### 2.2) Etablissement de lignées stables exprimant les protéines de fusion à partir du vecteur Dα.

Les vecteurs d'expression Dα-LAG-3_{(DID4)}Ig/E7 et Dα-LAG-3(_{DID4)}Ig/gagnef construits ont été transfectés dans des cellules CHO-*dhfr*⁻ en vue d'obtenir des lignées productrices stables exprimant les protéines recombinantes d'intérêt.

### 2.2.1) Méthode de transfection et d'isolement des clones.

### 2.2.1.1) Transfections

Les cellules CHO-*dbfr*⁻ (DSMZ ACC126), utilisées pour la transfection des constructions en vecteur Dα, sont cultivées en présence de ribonucléosides et désoxyribonucléosides (milieu MEMα RN/RdN+, GIBCO 22571-020). Les cellules CHO-*dhfr*⁻ ont été ensemencées la veille en flasques de 25cm2, et transfectées le 25 juillet 04 avec 1µg plasmide Da-LAG-3_{(DID4)}Ig/E7 ou Da-LAG-3_{(DID4)}Ig/gagnef, en utilisant 2,5 µl de lipofectamine 2000 (Invitrogen). Les transfections du vecteur d'origine Dα-LAG3-D1D4-ΔEK-hIgG1 Fusion et de pEGFP (Clontech) ont été utilisées comme contrôles positif et négatif, respectivement. Le milieu de transfection a été remplacé 6 heures après la transfection par du milieu MEMα RN/RdN+.

### 2.2.1.2) Sélection de cellules résistantes

Deux jours après transfection, les cellules ont été trypsinées et ensemencées pour chaque transfection en 4 plaques 96 puits à raison de 5000 cellules/puits en milieu de sélection MEMα RN/RdN- (GIBCO 22561-021). Après une semaine environ, le surnageant de chacun de ces puits, représentant chacun un "pool" de cellules, a été dosé en ELISA anti-LAG-3. Les "pools" les plus producteurs ont été amplifiés pour congélation et gardés en culture en présence de Methotrexate pour permettre l'amplification des séquences issues du plasmide (donc des gènes codant pour les protéines d'intérêt).

Toutes les étapes de sélection et d'expansion des pools et des clones ont été menées en milieu contenant du sérum de veau foetal sans endotoxine (GIBCO16000-044 et dialysé pour éviter l'apport de nucléosides par le sérum.

### 2.2.2) Sélection des meilleurs clones producteurs de protéine recombinante hLAG-3Ig/E7.

### 2.2.2.1) Sélection des "pools" de transfectants hLAG-3Ig/E7 les plus producteurs

Parmi les 400 "pools" testés en ELISA anti-LAG-3, 6 étaient bien meilleurs producteurs que les autres et ont été sélectionnés (pool#3, 4, 9, 11, 20, 21). Deux ampoules de chacun de ces "pools" ont été congelées.

### 2.2.2.2) Amplification génique par Methotrexate

Ces "pools" ont été ensemencés en plaque 6 puits à raison de 150000 cellules/puits et cultivés en présence de 50nM de Methotrexate. La quantité de hLAG-3Ig/E7 dans les surnageants des "pools" 9 et 11 était augmentée en présence de Methotrexate. Les doses de Methotrexate ont donc été augmentées à 150 et 250nM.

Le "pool" 9 résistait à 250nM Methotrexate, et la quantité de hLAG-3Ig/E7 produite était plus importante. Une expérience de dilution limite a donc été effectuée dans ces conditions le 11 septembre 04. Deux clones issus de ce "pool" ont été retenus (#9-23 et #9-26), et congelés le 8 octobre 04.

Le "pool" 11 résistait à 250nM Methotrexate, et la quantité de hLAG-31g/E7 produite était plus importante. Une expérience de dilution limite a donc été effectuée dans ces conditions le 3 septembre 04. Cinq clones issus de ce "pool" ont été retenus (#11-1, #11-2, #11-5, #11-6, #11-10) et congelés le 6 octobre 04.

L'adaptation de ces clones en milieux chimiquement définis (contenant une faible teneur en protéines exogènes) et supplémentés de 2mM de Butyrate (agent différenciant connu pour augmenter la production des protéines recombinantes) a été évaluée en termes de production de la protéine hLAG-3Ig/E7. Sur la base de son niveau de production dans les milieux sans sérum, de la qualité de la protéine produite (western blot et révélation par un anticorps anti-LAG-3) et de sa capacité proliférative, le clone hLAG-3Ig/E7 #11-5 a été sélectionné. Vingt ampoules de ce clone ont été congelées le 5 novembre 04.

### 2.2.3) Sélection des meilleurs clones producteurs des protéines recombinantes hLAG-3Ig/gagnef.

### 2.2.3.1) Sélection des "pools" de transfectants hLAG-3Ig/gagnef les plus producteurs

Parmi les 400 "pools" testés en ELISA anti-LAG-3, 24 ont été sélectionnés ("pools" numérotés de 1 à 24) et congelés.

### 2.2.3.2) Amplification génique par Methotrexate

Ces "pools" ont été ensemencés en plaque 6 puits à raison de 150000 cellules/puits et cultivés en présence de 50nM de Methotrexate. Onze pools résistant à 50nM Methotrexate ont été cultivés avec 150 et 250nM de Methotrexate.

Le "pool" 1 résistait à 250nM Methotrexate et la quantité de hLAG-3Ig/gagnef produite était plus importante. Une expérience de dilution limite a donc été effectuée dans ces conditions le 6 septembre 04. Cinq clones issus de ce "pool" ont été retenus (#1-14, #1-21, #1-49, #1-56, #1-100) et congelés le 6 octobre 04.

Le "pool" 6 résistait à 250nM Methotrexate et la quantité de hLAG-3Ig/gagnef produite était plus importante. Une expérience de dilution limite a donc été effectuée dans ces conditions le 11 septembre 04. Cinq clones ont été retenus (#6-15, #6-55, #6-57, #6-58; #6-65) et congelés le 5 octobre 04.

L'adaptation de ces clones en milieux chimiquement définis supplémentés de 2mM de Butyrate a été évaluée en termes de production de la protéine hLAG-3Ig/gagnef. Sur la base de sa productivité dans ces milieux sans sérum, de la qualité de la protéine produite (western blot et révélation par un anticorps anti-LAG-3) et de sa capacité proliférative, le clone hLAG-3Ig/gagnef #1-21 a été sélectionné. Vingt ampoules de ce clone ont été congelées le 29 novembre 04.

### 3) Production et purification des protéines de fusion

Les protéines hLAG-3Ig-E7, hLAG-3Ig/E7 et hLAG-3Ig/gagnef et le contrôle hLAG-3Ig ont été produites et purifiées.

### 3.1) Production des protéines de fusion

### 3.1.1) Production de grands volumes de surnageant à partir des lignées productrices de hLAG-3Ig-E7 et hLAG-3Ig

Les lignées productrices des protéines hLAG-3_{(D1D4)}Ig (CHO 7-1-B4), hLAG-3_{(D1D4)}Ig-E7 (CHO 7-1-D3), hLAG-3_{(D1D2)}Ig (CHO 21-1-C8) et hLAG-3_{(D1D2)}Ig-E7 (CHO 14-1-E11), ont été cultivées à plus grande échelle afin d'obtenir des volumes de surnageant contenant quelques milligrammes de protéine (entre 1.3 et 1.6 litres de surnageant par protéine).

Toutes les cultures ont été réalisées avec des cellules adhérentes cultivées en flasques de 175 cm2 avec 80 ml de milieu par flasque. Le milieu utilisé est du Ham F12 (Invitrogen), complété avec 10% de SVF (lot S135), sans antibiotique de sélection.

Les cellules ont été passées soit au 1/10° et laissées en culture 4 jours, soit au 1/3 et laissées en culture 2 jours. La récolte de surnageant a donc été faite avant que le milieu ne devienne trop jaune et que les cellules ne se décollent.

La concentration des lots de production en protéines recombinantes est de :
- hLAG-3_{(D1D4})Ig (CHO 7-1-B4) : 1.4 mg/l
- hLAG-3_{(D1D4)}Ig-E7 (CHO 7-1-D3) : 0.33 mg/l
- hLAG-3_{(D1D2)}Ig (CHO 21-1-C8) : 4 µg/l
- hLAG-3_{(D1D2)}Ig-E7 (CHO 14-1-E11) : 5 µg/l

La production des protéines recombinantes contenant seulement 2 domaines Ig de hLAG-3 est donc beaucoup moins efficace que celle avec 4 domaines Ig de hLAG-3.

### 3.1.2) Production de grands volumes de surnageant à partir des clones hLAG-3Ig/E7#11-5 et hLAG-3Ig/gagnef#1-21

L'augmentation de la biomasse des clones hLAG-3Ig/E7#11-5 et hLAG-3Ig/gagnef#1-21 a été faite en adhérence en présence de sérum. A confluence les cellules été lavées avec du PBS et cultivées en milieux ProCHO4-CDM (Cambrex BE12-029Q) supplémenté de 250nM Methotrexate et 2mM Butyrate à 30°C. Le milieu était récupéré toutes les 24 ou 36 heures. Plusieurs productions ont ainsi été effectuées et les quantités produites dans les surnageants étaient généralement supérieures à 2 mg/l pour la protéine de fusion hLAG-3Ig-E7 et à 1mg/l pour hLAG-3Ig-gagnef dans ce système d'expression.

### 3.2) Purification de hLAG-3Ig-E7 et hLAG-3Ig, hLAG-3Ig/E7 et hLAG-3Ig/gagnef

Les protéines recombinantes hLAG-3_{(D1D4)}Ig, hLAG-3_{(D1D4)}Ig-E7, hLAG-3_{(D1D2)}Ig, hLAG-3_{(D1D2)}Ig-E7, hLAG-3Ig/E7 et hLAG-3Ig/gagnef ont été purifiées sur colonne de protéine A, à partir des lots de surnageants produits.

### 3.2.1) Protocole de purification utilisé

La purification des protéines recombinantes à partir du surnageant de culture se fait sur colonne de protéine A (Pharmacia ref-17-5079-01) équilibrée en PBS.

Le surnageant de culture est passé sur filtre 0.22 µm. Il est chargé sur la colonne de protéine A, préalablement équilibrée en PBS, à l'aide d'une FPLC Pharmacia. Lors de la purification de hLAG-3_{(D1D4)}Ig, hLAG-3_{(D1D4)}Ig-E7, hLAG-3_{(D1D2})Ig, hLAG-3_{(D1D2)}Ig-E7, les protéines ne fixant pas la protéine A sont lavées avec 10 ml de PBS, puis la protéine recombinante est éluée grâce à un gradient de tampon glycine 0.1 M (pH compris entre 4 et 2.7). Des fractions de 1 ml sont collectées.

Le détecteur UV indique la présence d'un pic d'élution lorsque le gradient atteint environ 20% de tampon pH 2,7. Le profil d'élution était le même pour les quatre protéines recombinantes.

Les fractions contenant la protéine purifiée ont été mélangées, puis passées dans un tampon PBS sur colonne de déssalage (Hi-trap desalting 5 ml de Pharmacia). Les protéines ainsi obtenues ont (avant leur utilisation dans les tests fonctionnels) été concentrées sur concentrator (Vivascience réf.V50201 cut off 10kDa) et stérilisées par filtration sur colonne SpinX (réf. Costar 8160). Le produit a été aliquoté et congelé à -80°C.

Dans le cas des protéines recombinantes hLAG-3Ig/E7 et hLAG-3Ig/gagnef, après lavage en PBS, et en tampon glycine 0.1M pH4, la protéine est directement éluée en tampon glycine 0,1M pH2,7, puis dialysée contre du PBS, filtrée à 0,2µm, aliquotée et stockée à -80°C.

### 3.2.2) Rendement des étapes de purification

Le produit de chaque étape de la purification, du déssalage et de la concentration sur Centricon a été analysé par ELISA anti-hLAG-3Ig.

Le tableau 1 récapitule les concentrations obtenues par dosage ELISA, et des rendements de chaque étape de purification pour les protéines recombinantes hLAG-3_{(D1D4)}Ig, hLAG-3_{(D1D4)}Ig-E7, hLAG-3(_{D1D2)}Ig et hLAG-3_{(D1D2)}Ig-E7.

**Tableau 1**

| | | Surnageant | Eluat | lavage | Fractions 7-8-9 | Fractions 10-11-12 | Fractions 13-14 | Rendement |
|---|---|---|---|---|---|---|---|---|
| hLAG-3_{(D1D4)}Ig | Concentration (µg/ml) | 1,45 | 0,02 | 0 | 352 | 191 | 45,2 | 78.3% |
| | Quantité totale (µg) | 2169 | 31,8 | 0 | 1698.6 | | | |
| | Après déssalage | | / | | 170 µg/ml (pour 12ml) | | | 10% |
| | Après concentration | | | | 111 µg/ml (pour 12ml) | | | |
| hLAG-3_{(D1D4)}Ig -E7 | | 0,33 | 0,02 | 0 | 45,2 | 67,2 | 35,2 | 75,8% |
| | Quantité totale (µg) | 514,6 | 34,8 | 0 | 390 | | | |
| | Après déssalage (µg/ml) | | / | | 39 µg/ml (pour 12ml) | | | 25% |
| | Après concentration (µg/ml) | | | | 84 µg/ml (pour 12ml) | | | |
| hLAG-3_{(D1D2)}Ig | Concentration (µg/ml) | 0,004 | 0,003 | 0 | <0,25 | <0,25 | <0,25 | / |
| E7-hLAG-3_{(D1D2)}Ig | Concentration (µg/ml) | 0,006 | 0,006 | 0 | <0,25 | <0,25 | <0,25 | / |

Le rendement obtenu lors de la purification de hLAG-3_{(D1D4)}Ig et hLAG-3_{(D1D4})Ig-E7 sur colonne de protéine A est de l'ordre de 77%, ce qui est relativement satisfaisant.

La dilution du produit d'un facteur 1,5 lors du changement de tampon sur colonne de déssalage est normale.

Le tableau 2 récapitule les quantités obtenues par dosage protéique BCA (Perbio), ainsi que la teneur en Endotoxines estimées par LAL (Cambrex) dans les protéines hLAG-3Ig/E7 et hLAG-3Ig/gagnef purifiées.

**Tableau 2**

| hLAG-3Ig/ E7 | Date de purification | Quantité de protéines purifiée (mg) | Taux d'Endotoxines (EU/mg) |
|---|---|---|---|
| | 16 novembre 04 | 1.8 | 1.3 |
| | 1 décembre 04 | 2.8 | 1.5 |
| | 11 janvier 05 | 1.08 | 0.5 |
| | 28-janvier 05 | 2.40 | 1.4 |

| hLAG-3Ig/gagnef | Date de purification | Quantité de protéines purifiée (mg) | Taux d'Endotoxines (EU/mg) |
|---|---|---|---|
| | 10 décembre 04 | 1.25 | 0.3 |
| | 10 janvier 05 | 2.11 | 0.43 |
| | 21-janvier 05 | 1.60 | 0.65 |

L'utilisation du milieu sans sérum a permis de réduire le taux d'Endotoxines bien en dessous des limites imposées pour l'utilisation de notre invention *in vitro* sur les cellules dendritiques et chez l'animal.

### 3.2.3) Analyse des produits purifiés sur gel SDS-PAGE

La nature et la pureté des produits de purification des quatre protéines recombinantes hLAG-3_{(D1D4)}Ig, hLAG-3(_{D1D4})Ig-E7, hLAG-3_{(D1D2)}Ig et hLAG-3_{(D1D2)}Ig-E7, a été analysée sur gel SDS-PAGE. Les gels à 10% d'acrylamide ont été, soit colorés au bleu de Coomassie, soit transférés sur nitrocellulose pour analyse par western blot anti-hLAG3 et anti-E7 (exemples en figure 5).

La protéine hLAG-3_{(D1D4)}Ig purifiée sur une colonne de protéine A migre de la même façon que la protéine hLAG-3_{(D1D4)}Ig purifiée (figure 5). Par contre, deux protéines de taille inférieure sont aussi présentes en grande quantité dans notre produit de purification. Elles correspondent aux immunoglobulines bovines présentes dans le sérum utilisé pour la culture des cellules.

hLAG-3_{(D1D4)}Ig fusionnée à E7 migre légèrement moins vite, ce qui est attendu. Afin de confirmer la présence du fragment E7, un Western blot avec un anticorps anti-E7 a été réalisé. La fusion hLAG-3_{(D1D4)}Ig-E7 est révélée.

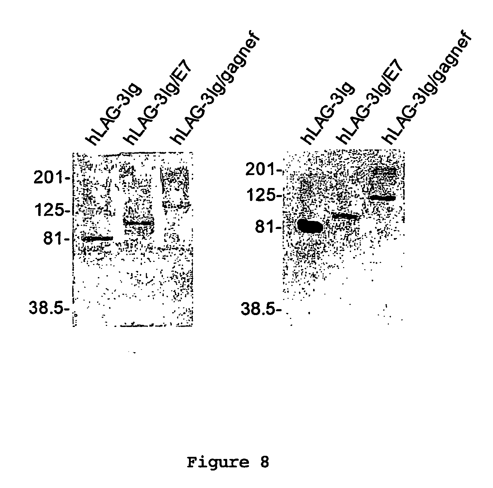

La nature et la pureté des produits de purification de chaque lot de hLAG-3_{(D1D4)}I9/-E7, hLAG-3_{(D1D4)}Ig/gagnef ont été analysées par coloration au bleu de Coomassie et Western blot anti-LAG-3 (exemple en figure 8).

Comme attendu, les protéines hLAG-3_{(D1D4)}Ig/E7 et hLAG-3_{(D1D4)}Ig/gagnef purifiées présentent un poids moléculaire apparent supérieur à hLAG-3_{(D1D4)}Ig. Le fragment gagnef étant plus grand que le fragment E7, la protéine de fusion hLAG-3(_{D1D4}) Ig/gagnef présente la migration la plus lente (figure 8). L'utilisation du milieu sans sérum a permis d'éliminer les protéines contaminantes.

### 4) Test de la fonctionnalité des protéines de fusion

Les protéines hLAG-3_{(D1D4)}Ig, hLAG-3_{(D1D4)}Ig-E7, hLAG-3_{(D1D4)}Ig/E7 et hLAG-3_{(D1D4)}Ig/gagnef ainsi purifiées ont été testées pour leur capacité à fixer le CMH de classe II, pour leur capacité à induire la maturation des cellules dendritiques, à être internalisée dans des cellules dendritiques et leur capacité à induire une réponse T CD4 et/ou CD8 spécifique.

### 4.1) Fixation au CMH de classe II

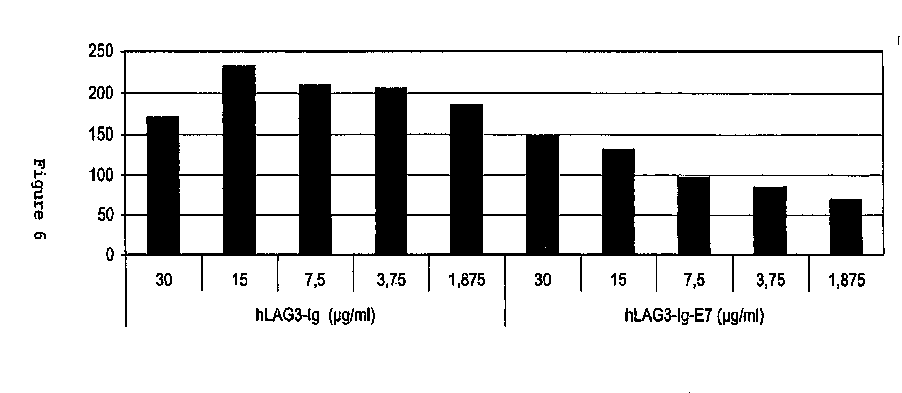

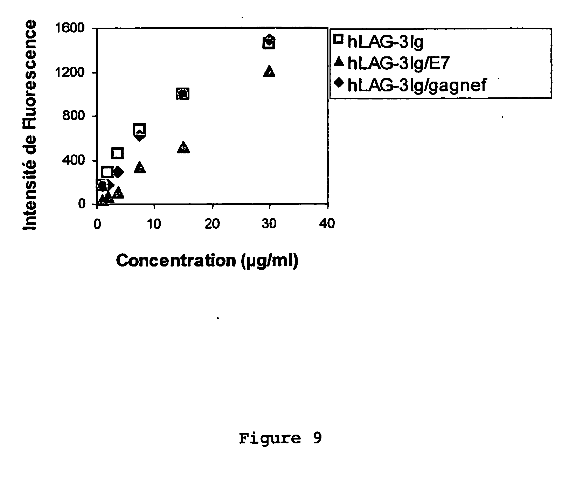

La capacité des protéines recombinantes à lier le CMH de classe II a été évaluée en mesurant la fixation des protéines sur des cellules LAZ-509 (cellules B humaines transformées par EBV, exprimant fortement le CMH de classe II). La fixation a été révélée grâce à un anticorps anti-Ig humaines couplé au FITC. L'intensité de marquage FITC des cellules LAZ-509, proportionnelle à la fixation de la protéine recombinante sur le CMH de classe II, a été quantifiée par analyse au FACS (figure 6 et 9).

La protéine de fusion entre hLAG-3_{(D1D4)}Ig et E7 se fixe au CMH de classe II de façon comparable à hLAG-3_{(D1D4})Ig produite et purifiée dans les mêmes conditions (figure 6).

De même, la capacité de liaison des protéines hLAG-3_{(D1D4)}Ig/E7 et hLAG-3_{(D1D4)}Ig/gagnef est similaire à celle de hLAG-3Ig (figure 9).

### 4.2) Maturation des cellules dendritiques humaines par hLAG-3_{(D1D4)}Ig/E7 et hLAG-3_{(D1D4)}Ig/gagnef

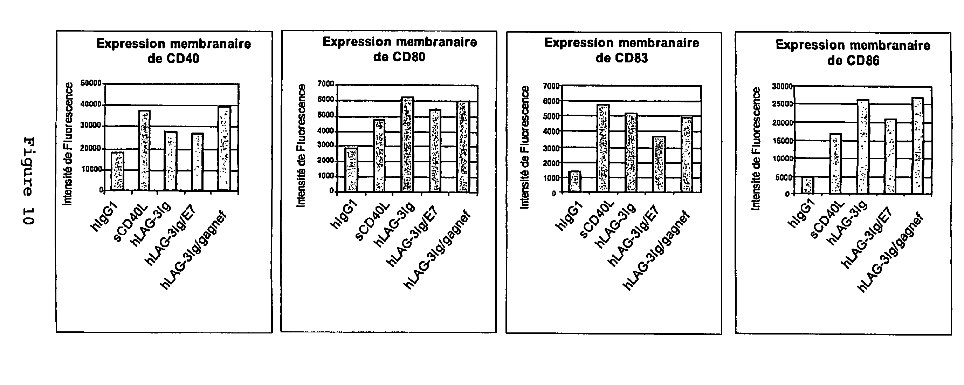

Les cellules dendritiques immatures différenciées à partir de monocytes de sang périphérique sont incubées pendant 2 jours avec des IgGl humaines (comme contrôle négatif de stimulation) ou les protéines hLAG-3_{(D1D4)}Ig/E7, hLAG-3_{(D1D4)}Ig/gagnef ou hLAG-3Ig (10µg/ml). Soluble CD40-ligand (sCD40L, 3µg/ml) connu pour induire la maturation des cellules dendritiques était utilisé comme contrôle positif de stimulation. L'activation des cellules dendritiques est alors évaluée par l'expression membranaire des marqueurs d'activation CD40, CD80, CD83, CD86 (exemple en figure 10). Les protéines de fusion LAG-3/E7 ou LAG-3/gagnef induisent la maturation des cellules dendritiques tout comme les deux contrôles positifs, LAG-3Ig et sCD40L.

### 4.3) Internalisation dans des cellules dendritiques humaines de hLAG-3Ig-E7

L'internalisation de la protéine de fusion entre hLAG-3_{(D1D4)}Ig et E7 dans les cellules dendritiques a été testée.

Des cellules dendritiques humaines immatures purifiées à partir de PBMC ont été incubées à 4°C en présence des protéines recombinantes à 30µg/ml. Ces cellules sont ensuite placées 15 minutes à 37°C. Nous savons que dans ces conditions hLAG3-Ig est internalisé dans les cellules dendritiques et induit leur maturation.

Une analyse au microscope confocal a permis de révéler que la protéine hLAG-3_{(D1D4)}Ig-E7 est internalisée efficacement dans les cellules dendritiques immatures humaines (figure 10). Après avoir mis les lames 15 min à 37°C, les cellules internalisent la protéine LAG-3Ig-E7 avec un marquage spécifique dans les cellules dendritiques, qui n'est pas retrouvé à 4°C (contrôle négatif des l'internalisation).

### 4.4) Réponses T CD4 et T CD8 à l'antigène E7 et à l'antigène gag-nef

Des cellules PBMC sont fraîchement collectées à partir de donneurs sains, et purifiées en utilisant un Ficoll-Paque standard (Amercsham Pharmacia Biotech AB).

Les cellules dendritiques sont préparées en cultivant des PBMC en présence de 500 U/ml de GM-CSF(R&D Systems Inc. MN) et 500 U/ml d'IL-4 (R&D Systems Inc. MN) pendant 7 jours.

Les cellules dendritiques sont ensuite maturées pendant 2 jours en présence de 2 µg/ml d'anticorps anti-CD40 et 100 ng/ml de Poly IC (Sigma Aldrich).

Les cellules T CD4 et CD8 purifiées sont stimulées chaque semaine avec des cellules dendritiques pendant 2 heures avec les protéines hLAG-3_{(D1D4)}Ig/E7, hLAG-3_{(D1D4)}Ig/gagnef, E7, gagnef ou hLAG-3Ig (10µg/ml) et irradiées à 35 Gy avec un rapport cellules T/cellules dendritiques de 10/1 dans le milieu de culture (CM ;RPMI 1640 avec 10 sérum AB humain, 10nM L-Glutamine et de la gentamycine). 1.10³U/ml d'IL-6 et 5 U/ml d'IL-12 (R&D Systems Inc. MN) sont ajoutés lors de la première semaine de culture. 20 U/ml d'IL-2 (R&D Systems Inc. MN) et 10 ng/ml d'IL-7 (R&D Systems Inc. MN) sont ajoutés lors des deux semaines suivantes.

Les essais Elispot sont utilisés afin de quantifier les cellules effectrices sécrétant l'interféron-γ en réponse aux antigènes E7 ou gag-nef.

Des plaques de 96 puits de membrane d'ester de cellulose (MultiScreen MAHA 54510 ; Millipore) sont recouverts pendant une nuit par des anticorps anti-interféron-γ (MAB 285). Les puits sont lavés et couverts par du milieu de culture à 10 % de sérum humain AB, et les cellules sont ajoutées à quatre concentrations différentes. Les protéines sont ajoutées dans chaque puits et les plaques sont incubées toute la nuit. Le jour suivant, le milieu est éliminé et les puits sont lavés avant l'addition d'un anticorps secondaire biotinylé (BAF285-Biotin). Les plaques sont incubées pendant 2 heures et lavées et le complexe streptavidine-enzyme (Straptavidine-AP ; Boehringer Mannheim GmbH) est ajouté dans chaque puits. Les plaques sont incubées à température ambiante pendant 1 heure, et le substrat BCIP-NBT de l'enzyme (S3771 : Proméga France) est ajouté dans chaque puits dans le tampon de la phosphatase alcaline à pH 9,5 (100mM tris HCl, 100mM NaCl, 5mM MgCl2) et les plaques sont incubées à température ambiante pendant 10 à 20 minutes. La réaction se termine par le lavage à l'eau dès l'émergence de spots violet foncé. Les spots sont comptés en utilisant un système d'analyse d'images automatique ELISPOT Reader (AID Strasbourg, Allemagne).

La fréquence des cellules effectrices T CD4 ou T CD8 sécrétant l'interféron-γ en réponse aux antigènes peut être calculée à partir du nombre de cellules formant des spots et à partir de la fréquence des cellules T CD8 ou CD4 dans une population de lymphocytes grâce à un immunomarquage avec un anticorps anti-CD8 ou anti-CD4.

## Revendications

1. Produit de couplage, **caractérisé en ce qu'**il est constitué d'une première classe de protéine de type antigène choisie dans le groupe comprenant des antigènes viraux, des antigènes bactériens, des antigènes tumoraux, des antigènes de parasites et leurs mélanges et d'une seconde classe de protéine de type ligand du CMH de classe II choisie dans le groupe comprenant hLAG-3, ses homologues, fragments et dérivés et les mélanges de ceux-ci, les deux classes de protéine étant couplées par une ou plusieurs liaisons stables dans les milieux biologiques, lesdits homologues, fragments et dérivés étant capables d'assurer une fixation avec une forte affinité sur le CMH de classe **II** des cellules dendritiques et l'internalisation de l'antigène de ladite première classe de protéine.

2. Produit de couplage selon la revendication 1, **caractérisé en ce que** les deux classes de protéine sont liées par covalence.

3. Produit de couplage selon la revendication 2, **caractérisé en ce que** les deux classes de protéine sont liées par covalence indirectement via un bras de liaison ou une molécule de liaison.

4. Produit de couplage selon l'une des revendications 2 ou 3, **caractérisé en ce que** les deux classes de protéine forment une protéine de fusion.

5. Produit de couplage selon la revendication 1, **caractérisé en ce que** le fragment de LAG-3 est un fragment soluble.

6. Produit de couplage selon la revendication 5, **caractérisé en ce que** le fragment de LAG-3 est choisi dans le groupe comprenant les fragments D1-D2 et D1-D4.

7. Produit de couplage selon l'une quelconque des revendications précédentes, **caractérisé en ce que** la première classe de protéine de type antigène est choisie dans le groupe comprenant des antigènes spécifiques d'une pathologie dont la thérapie nécessite une réponse lymphocytaire T.

8. Produit de couplage selon la revendication 1, **caractérisé en ce que** la première classe de protéine de type antigène est un antigène viral choisi dans le groupe comprenant les virus HPV, HBV, HCV, HIV, EBV, CMV et leurs mélanges.

9. Produit de couplage selon la revendication 8, **caractérisé en ce que** la première classe de protéine de type antigène est choisi dans le groupe comprenant l'antigène E7 d'HPV et l'antigène gag-nef d'HIV.

10. Produit de couplage selon la revendication 1, **caractérisé en ce que** la première classe de protéine de type antigène est un antigène bactérien choisi dans le groupe des bactéries intracellulaires de la tuberculose, de la lèpre, de la listéria.

11. Produit de couplage selon la revendication 1, **caractérisé en ce que** la première classe de protéine de type antigène est un antigène tumoral choisi dans le groupe comprenant CEA, Melan A, PSA, MAGE-3, HER2/neu, protéine E6 et E7 d'HPV.

12. Composition de vaccin **caractérisée en ce qu'**elle comprend au moins un produit de couplage selon l'une quelconque des revendications précédentes, éventuellement associé à un véhicule pharmaceutiquement acceptable.

13. Utilisation d'un produit de couplage selon l'une quelconque des revendications 1 à 11 pour la préparation d'un médicament destiné au traitement des maladies infectieuses et/ou du cancer.

14. Produit de couplage selon l'une quelconque des revendications 1 à 11 pour utilisation dans le traitement des maladies infectieuses et/ou du cancer.

15. Utilisation selon l'une des revendications 13 ou 14, dans laquelle le traitement des maladies infectieuses et/ou du cancer implique une réponse Immunitaire via les cellules T CD8+.

16. Utilisation selon l'une quelconque des revendications 13 à 15, dans laquelle la seconde classe de protéine de type ligand du CMH II est capable d'induire une réponse immunitaire antigène spécifique via les cellules T.

17. Utilisation d'un produit de couplage selon l'une quelconque des revendications 1 à 11 pour la fabrication d'un médicament destiné à l'immunothérapie des cancers et/ou l'immunothérapie des maladies infectieuses.

18. Produit de couplage selon l'une quelconque des revendications 1 à 11 pour utilisation dans l'immunothérapie des cancers et/ou l'immunothérapie des maladies infectieuses.

19. Utilisation d'un produit de couplage selon l'une quelconque des revendications 1 à 11 pour la préparation d'une composition immunogène capable d'induire une réponse T CD4 et/ou CD8 spécifique.

20. Produit de couplage selon l'une quelconque des revendications 1 à 11 pour utilisation à induire une réponse T CD4 et/ou CD8 spécifique.

## Claims

1. Coupling product, **characterised in that** it is formed of a first class of protein of the antigen type selected from the group comprising viral antigens, bacterial antigens, tumour antigens, parasitic antigens, and mixtures thereof, and a second class of protein of the class II MHC ligand type selected from the group comprising hLAG-3, its homologues, fragments and derivatives, and mixtures thereof, the two classes of protein being coupled by one or more bonds which are stable in biological environments, said homologues, fragments and derivatives being able to ensure fixation with a strong affinity on the class II MHC of dendritic cells and internalisation of the antigen of said first class of protein.

2. Coupling product according to claim 1, **characterised in that** the two classes of protein are linked by covalent bonds.

3. Coupling product according to claim 2, **characterised in that** the two classes of protein are indirectly linked by covalent bonds via a linker or a linking molecule.

4. Coupling product according to either claim 2 or claim 3, **characterised in that** the two classes of protein form a fusion protein.

5. Coupling product according to claim 1, **characterised in that** the LAG-3 fragment is a soluble fragment.

6. Coupling product according to claim 5, **characterised in that** the LAG-3 fragment is selected from the group comprising D1-D2 and D1-D4 fragments.

7. Coupling product according to any one of the preceding claims, **characterised in that** the first class of protein of the antigen type is selected from the group comprising antigens specific to a disease, the treatment of which requires a T lymphocyte response.

8. Coupling product according to claim 1, **characterised in that** the first class of protein of the antigen type is a viral antigen selected from the group comprising the HPV, HBV, HCV, HIV, EBV, CMV viruses and mixtures thereof.

9. Coupling product according to claim 8, **characterised in that** the first class of protein of the antigen type is selected from the group comprising the antigen E7 of HPV and the gag-nef antigen of HIV.

10. Coupling product according to claim 1, **characterised in that** the first class of protein of the antigen type is a bacterial antigen selected from the group of intracellular bacteria of tuberculosis, leprosy and listeria.

11. Coupling product according to claim 1, **characterised in that** the first class of protein of the antigen type is a tumour antigen selected from the group comprising CEA, Melan A, PSA, MAGE-3, HER2/neu, and E6 and E7 proteins of HPV.

12. Vaccine composition **characterised in that** it comprises at least one coupling product according to any one of the preceding claims, optionally combined with a pharmaceutically acceptable excipient.

13. Use of coupling product according to any one of claims 1 to 11 for the preparation of a drug intended for treating infectious diseases and/or cancer.

14. Coupling product according to any one of claims 1 to 11 for use in the treatment of infectious diseases and/or cancer.

15. Use according to either claim 13 or claim 14, in which the treatment of infectious diseases and/or cancer implies an immune response via CD8+ T cells.

16. Use according to any one of claims 13 to 15, in which the second class of protein of the MHC II ligand type is capable of inducing an antigen-specific immune response via the T cells.

17. Use of coupling product according to any one of claims 1 to 11, for the manufacture of a drug intended for the immunotherapy of cancers and/or the immunotherapy of infectious diseases.

18. Coupling product according to any one of claims 1 to 11 for use in the immunotherapy of cancers and/or the immunotherapy of infectious diseases.

19. Use of a coupling product according to any one of claims 1 to 11 for the preparation of an immunogenic composition capable of inducing a specific CD4 and/or CD8 T-cell response.

20. Coupling product according to any one of claims 1 to 11 for use to induce a specific CD4 and/or CD8 T-cell response.

## Patentansprüche

1. Kopplungsprodukt, **dadurch gekennzeichnet, dass** es aus einer ersten Proteinklasse vom Typ Antigen, die aus der Gruppe bestehend aus Viren-Antigenen, Bakterien-Antigenen, Tumor-Antigenen, Parasiten-Antigenen und ihren Mischungen ausgewählt ist, und aus einer zweiten Proteinklasse vom Typ MHC-Klasse-II-Ligand, die aus der Gruppe bestehend aus hLAG-3, seinen Homologen, Fragmenten und Abkömmlingen sowie Mischungen derselben ausgewählt ist, gebildet ist, wobei die beiden Proteinklassen über eine oder mehrere Verbindungen gekoppelt sind, die in biologischen Umgebungen stabil sind, wobei die Homologe, Fragmente und Abkömmlinge in der Lage sind, eine Bindung mit hoher Affinität am MHC der Klasse II von dendritischen Zellen und die Internalisierung des Antigens der ersten Proteinklasse zu gewährleisten.

2. Kopplungsprodukt nach Anspruch 1, **dadurch gekennzeichnet, dass** die beiden Proteinklassen über kovalente Bindungen verbunden sind.

3. Kopplungsprodukt nach Anspruch 2, **dadurch gekennzeichnet, dass** die beiden Proteinklassen über kovalente Bindungen indirekt über einen Linker oder ein Verbindungsmolekül verbunden sind.

4. Kopplungsprodukt nach Anspruch 2 oder 3, **dadurch gekennzeichnet, dass** die beiden Proteinklassen ein Fusionsprotein bilden.

5. Kopplungsprodukt nach Anspruch 1, **dadurch gekennzeichnet, dass** das LAG-3-Fragment ein lösliches Fragment ist.

6. Kopplungsprodukt nach Anspruch 5, **dadurch gekennzeichnet, dass** das LAG-3-Fragment aus der Gruppe bestehend aus den Fragmenten D1-D2 und D1-D4 ausgewählt ist.

7. Kopplungsprodukt nach einem der vorhergehenden Ansprüche, **dadurch gekennzeichnet, dass** die erste Proteinklasse vom Typ Antigen aus der Gruppe bestehend aus spezifischen Antigenen für eine Erkrankung ausgewählt ist, bei deren Behandlung eine Antwort der T-Lymphozyten erforderlich ist.

8. Kopplungsprodukt nach Anspruch 1, **dadurch gekennzeichnet, dass** die erste Proteinklasse vom Typ Antigen ein Viren-Antigen ist, das aus der Gruppe bestehend aus den Viren HPV, HBV, HCV, HIV, EBV, CMV und ihren Mischungen ausgewählt ist.

9. Kopplungsprodukt nach Anspruch 8, **dadurch gekennzeichnet, dass** die erste Proteinklasse vom Typ Antigen aus der Gruppe bestehend aus dem HPV-Antigen E7 und dem HIV-Antigen gag-nef ausgewählt ist.

10. Kopplungsprodukt nach Anspruch 1, **dadurch gekennzeichnet, dass** die erste Proteinklasse vom Typ Antigen ein Bakterien-Antigen ist, das aus der Gruppe bestehend aus intrazellulären Tuberkulosebakterien, Leprabakterien und Listerien ausgewählt ist.

11. Kopplungsprodukt nach Anspruch 1, **dadurch gekennzeichnet, dass** die erste Proteinklasse vom Typ Antigen ein Tumor-Antigen ist, das aus der Gruppe bestehend aus CEA, Melan A, PSA, MAGE-3, HER2/neu, und den HPV-Proteinen E6 und E7 ausgewählt ist.

12. Impfstoffzusammensetzung, **dadurch gekennzeichnet, dass** sie mindestens ein Kopplungsprodukt nach einem der vorhergehenden Ansprüche umfasst, gegebenenfalls verbunden mit einem pharmazeutisch akzeptablen Träger.

13. Verwendung eines Kopplungsprodukts nach einem der Ansprüche 1 bis 11 zur Herstellung eines Medikaments, das für die Behandlung von Infektionskrankheiten und/oder von Krebs bestimmt ist.

14. Kopplungsprodukt nach einem der Ansprüche 1 bis 11 zur Verwendung bei der Behandlung von Infektionskrankheiten und/oder von Krebs.

15. Verwendung nach einem der Ansprüche 13 oder 14, wobei die Behandlung von Infektionskrankheiten und/oder von Krebs eine Immunantwort über die CD8+-T-Zellen beinhaltet.

16. Verwendung nach einem der Ansprüche 13 bis 15, wobei die zweite Proteinklasse vom Typ MHC-II-Ligand in der Lage ist, eine antigenspezifische Immunantwort über die T-Zellen auszulösen.

17. Verwendung eines Kopplungsprodukts nach einem der Ansprüche 1 bis 11 zur Herstellung eines Medikaments, das für die Immuntherapie von Krebserkrankungen und/oder die Immuntherapie von Infektionskrankheiten bestimmt ist.

18. Kopplungsprodukt nach einem der Ansprüche 1 bis 11 zur Verwendung bei der Immuntherapie von Krebserkrankungen und/oder der Immuntherapie von Infektionskrankheiten.

19. Verwendung eines Kopplungsprodukts nach einem der Ansprüche 1 bis 11 zur Herstellung einer immunogenen Zusammensetzung, die in der Lage ist, eine spezifische CD4- und/oder CD8-T-Antwort auszulösen.

20. Kopplungsprodukt nach einem der Ansprüche 1 bis 11 zur Verwendung beim Auslösen einer spezifischen CD4- und/oder CD8-T-Antwort.


### SEQUENCE LISTING

<110> IMMUTEP
<120> composition de vaccin comprenant un ligand CMH de classe II couplé à un antigène, procédé de préparation et utilisations
<130> 35732/PCT
<140> PCT/FR05/XXXXX
   <141> 2005-04-13
<150> FR 04/03848
   <151> 2004-04-13
<160> 21
<170> PatentIn version 3.1
<210> 1
   <211> 35
   <212> DNA
   <213> Artificial sequence
<220>
   <223> amorce PCR
<400> 1
   ctgatctcta cggttatggg caattaaatg acagc 35
<210> 2
   <211> 35
   <212> DNA
   <213> Artificial séquence
<220>
   <223> amorce PCR
<400> 2
   gctgtcattt aattgcccat aaccgtagag atcag 35
<210> 3
   <211> 29
   <212> DNA
   <213> Artificial sequence
<220>
   <223> amorce PCR
<400> 3
   ccgctcgaga tgcatggaga tacacctac 29
<210> 4
   <211> 27
   <212> DNA
   <213> Artificial sequence
<220>
   <223> amorce PCR
<400> 4
   gctctagatt atggtttctg agaacag 27
<210> 5
   <211> 28
   <212> DNA
   <213> Artificial sequence
<220>
   <223> amorce PCR
<400> 5
   ggaattcgcc cagaccatag gagagatg 28
<210> 6
   <211> 28
   <212> DNA
   <213> Artificial sequence
<220>
   <223> amorce PCR
<400> 6
   ccgctcgagt ttacccgggg acagggag 28
<210> 7
   <211> 28
   <212> DNA
   <213> Artificial sequence
<220>
   <223> amorce PCR
<400> 7
   ggcgcgccat gcatggagat acacctac 28
<210> 8
   <211> 27
   <212> DNA
   <213> Artificial sequence
<220>
   <223> amorce PCR
<400> 8
   ggggtacctg gtttctgaga acagatg 27
<210> 9
   <211> 26
   <212> DNA
   <213> Artificial sequence
<220>
   <223> amorce PCR
<400> 9
   ggggtaccct ccagccaggg gctgag 26
<210> 10
   <211> 27
   <212> DNA
   <213> Artificial sequence
<220>
   <223> amorce PCR
<400> 10
   ccgctcgagt catttacccg gggacag 27
<210> 11
   <211> 26
   <212> DNA
   <213> Artificial sequence
<220>
   <223> amorce PCR
<400> 11
   ggggtacccg agggtgagta ctaagc 26
<210> 12
   <211> 27
   <212> DNA
   <213> Artificial sequence
<220>
   <223> amorce PCR
<400> 12
   ccgctcgagt catttacccg gggacag 27
<210> 13
   <211> 27
   <212> DNA
   <213> Artificial sequence
<220>
   <223> amorce PCR
<400> 13
   ccgctcgagc tccagccagg ggctgag 27
<210> 14
   <211> 27
   <212> DNA
   <213> Artificial sequence
<220>
   <223> amorce PCR
<400> 14
   ccgctcgagt catttacccg gggacag 27
<210> 15
   <211> 27
   <212> DNA
   <213> Artificial sequence
<220>
   <223> amorce PCR
<400> 15
   ccgctcgagc gagggtgagt actaagc 27
<210> 16
   <211> 27
   <212> DNA
   <213> Artificial sequence
<220>
   <223> amorce PCR
<400> 16
   ccgctcgagt catttacccg gggacag 27
<210> 17
   <211> 10
   <212> PRT
   <213> Artificial sequence
<220>
   <223> polypeptide de liaison
<400> 17
   Asp Asp Asp Asp Lys Gly Ser Gly Ser Gly
   1               5                   10
<210> 18
   <211> 149
   <212> PRT
   <213> Artificial sequence
<220>
   <223> Domaine D1 de hHLAG-3
<400> 18
   Leu Gln Pro Gly Ala Glu Val Pro Val Val Trp Ala Gln Glu Gly Ala
   1 5 10 15
   Pro Ala Gln Leu Pro Cys Ser Pro Thr Ile Pro Leu Gln Asp Leu Ser
   20 25 30
   Leu Leu Arg Arg Ala Gly Val Thr Trp Gln His Gln Pro Asp Ser Gly
   35 40 45
   Pro Pro Ala Ala Ala Pro Gly His Pro Leu Ala Pro Gly Pro His Pro
   50 55 60
   Ala Ala Pro Ser Ser Trp Gly Pro Arg Pro Arg Arg Tyr Thr Val Leu
   65 70 75 80
   Ser Val Gly Pro Gly Gly Leu Arg Ser Gly Arg Leu Pro Leu Gln Pro
   85 90 95
   Arg Val Gln Leu Asp Glu Arg Gly Arg Gln Arg Gly Asp Phe Ser Leu
   100 105 110
   Trp Leu Arg Pro Ala Arg Arg Ala Asp Ala Gly Glu Tyr Arg Ala Ala
   115 120 125
   Val His Leu Arg Asp Arg Ala Leu Ser Cys Arg Leu Arg Leu Arg Leu
   130 135 140
   Gly Gln Ala Ser Met
   145
<210> 19
   <211> 90
   <212> PRT
   <213> Artificial sequence
<220>
   <223> Domaine D2 de hHLAG-3
<400> 19
   Thr Ala Ser Pro Pro Gly Ser Leu Arg Ala Ser Asp Trp Val Ile Leu
   1 5 10 15
   Asn Cys Ser Phe Ser Arg Pro Asp Arg Pro Ala Ser Val His Trp Phe
   20 25 30
   Arg Asn Arg Gly Gln Gly Arg Val Pro Val Arg Glu Ser Pro His His
   35 40 45
   His Leu Ala Glu Ser Phe Leu Phe Leu Pro Gln Val Ser Pro Met Asp
   50 55 60
   Ser Gly Pro Trp Gly Cys Ile Leu Thr Tyr Arg Asp Gly Phe Asn Val
   65 70 75 80
   Ser Ile Met Tyr Asn Leu Thr Val Leu Gly
   85 90
<210> 20
   <211> 91
   <212> PRT
   <213> Artificial sequence
<220>
   <223> Domaine D3 de hHLAG-3
<400> 20
   Leu Glu Pro Pro Thr Pro Leu Thr Val Tyr Ala Gly Ala Gly Ser Arg
   1 5 10 15
   Val Gly Leu Pro Cys Arg Leu Pro Ala Gly Val Gly Thr Arg Ser Phe
   20 25 30
   Leu Thr Ala Lys Trp Thr Pro Pro Gly Gly Gly Pro Asp Leu Leu Val
   35 40 45
   Thr Gly Asp Asn Gly Asp Phe Thr Leu Arg Leu Glu Asp Val Ser Gln
   50 55 60
   Ala Gln Ala Gly Thr Tyr Thr Cys His Ile His Leu Gln Glu Gln Gln
   65 70 75 80
   Leu Asn Ala Thr Val Thr Leu Ala Ile Ile Thr
   85 90
<210> 21
   <211> 82
   <212> PRT
   <213> Artificial sequence
<220>
   <223> Domaine D4 de hHLAG-3
<400> 21
   Val Thr Pro Lys Ser Phe Gly Ser Pro Gly Ser Leu Gly Lys Leu Leu
   1               5                   10                  15
   Cys Glu Val Thr Pro Val Ser Gly Gln Glu Arg Phe Val Trp Ser Ser
               20                  25                  30
   Leu Asp Thr Pro Ser Gln Arg Ser Phe Ser Gly Pro Trp Leu Glu Ala
           35                  40                  45
   Gln Glu Ala Gln Leu Leu Ser Gln Pro Trp Gln Cys Gln Leu Tyr Gln
       50                  55                  60
   Gly Glu Arg Leu Leu Gly Ala Ala Val Tyr Phe Thr Glu Leu Ser Ser
   65                  70                  75                  80
   Pro Gly